# EUROPEAN PATENT SPECIFICATION

(11) **EP 2 846 622 B1**
(45) Date of publication and mention of the grant of the patent: **01.02.2017**
(21) Application number: 13724880.3
(22) Date of filing: 05.04.2013
(51) Int. Cl.: A01H 4/00

(54) **PROPAGATION OF STRELITZIA (BIRD OF PARADISE)**
ZÜCHTUNG VON STRELITZIEN (PARADIESVOGELBLUME)
PROPAGATION DE STRELITZIA (OISEAU DE PARADIS)

(30) Priority: 05.04.2012 ZA 201202513
(43) Date of publication of application: 18.03.2015
(73) Proprietor: University of Limpopo, 0727 Sovenga (ZA)
(72) Inventor: NIKOLOVA, Roumiana, Vassileva, 0699 Polokwane (ZA); RAMALEPE, Phillemon, 0837 Madjadji (ZA); DU PLESSIS, Helena, Jacoba, Polokwane 0713 (ZA)
(74) Representative: Lee, Nicholas John
(86) International application number: PCT/IB2013/052728
(87) International publication number: WO 2013/150493

(56) References cited:
- JOSEÉ GERALDO BARBOSA ET AL: "EFFECT OF ACID SCARIFICATION AND DIFFERENT TEMPERATURES ON PHYSIOLOGICAL QUALITY OF Strelitzia reginae SEEDS", REVISTA BRASILEIRA DE SEMENTES, vol. 27, no. 1, 1 January 2005 (2005-01-01), pages 71-77, XP055070367, cited in the application
- "Bird of Paradise (Strelitzia)" In: HK Singh: "Flower Crops - Cultivation and Management", 2006, New India Publishing Agency, New Dehli, XP002700703, ISBN: 81-89422-35-9 pages 37-46, cited in the application page 44, paragraph 3 page 45, paragraph 3
- ZIV M [REPRINT AUTHOR] ET AL: "CONTROL OF OXIDATIVE BROWNING AND IN-VITRO PROPAGATION OF STRELITZIA -REGINAE", HORTSCIENCE, AMERICAN SOCIETY OF HORTICULTURAL SCIENCE, ALEXANDRIA, VA, US, vol. 18, no. 4, 1 January 1983 (1983-01-01), pages 434-436, XP009171036, ISSN: 0018-5345
- POL P A VAN DE ET AL: "Vegetative propagation of Strelitzia reginae", ACTA HORTICULTURAE, INTERNATIONAL SOCIETY FOR HORTICULTURAL SCIENCE, BE, vol. 2, no. 226, 1 January 1988 (1988-01-01), pages 581-586, XP009171011, ISSN: 0567-7572
- PATRICIA DUARTE DE OLIVEIRA PAIVA ET AL: "In vitro stablisment of strelitzia ( Strelitzia reginae Banks.)", CIÊNCIA E AGROTECNOLOGIA, vol. 28, no. 5, 1 January 2004 (2004-01-01), pages 1031-1037, XP055070371,

## Description

### FIELD OF INVENTION

The invention relates to the propagation of plants. More particularly, the invention relates to *in vitro* propagation of plants of the genus *Strelitzia.* Still more particularly, the invention relates to *in vitro* propagation of plants of the species *Strelitzia juncea.*

### BACKGROUND

*Strelitzia juncea* is a monocotyledonous plant endemic to South Africa and has a significant horticultural potential due to its drought-tolerance, striking appearance and national and international market demand. Since the plant is not easily propagated by seeds or vegetatively, due to slow growth and low multiplication rate, *in vitro* culture is an alternative method that can be applied to increase the multiplication rate under controlled conditions. However, despite attempts made on *in vitro* propagation of some *Strelitzia* species such as the popular S. *reginae,* no successful mass propagation has been achieved by tissue culture. Major constraints for *in vitro* culture of this species are oxidative browning due to the presence of high levels of phenolic compounds in plant tissues and the recalcitrant nature of the plant. Therefore, there was a need to determine the effect of various limiting factors on *in vitro* culture and optimise the conditions.

The species name *juncea* is derived from the Latin word *"juncus"* which means rush that refers to the rush-like appearance of mature plants. It is a perennial herb that can grow up to two metres high and produces thick, fleshy roots and flowers with three large orange sepals and three bright blue petals (Van der Walt, 2000). In its natural habitat, it is pollinated mainly by sunbirds and sugarbirds, but if it is growing in places where these pollinators do not exist, hand pollination of the flowers is necessary (Winter, 2004; Sayers, 2007). It produces fruit capsules containing numerous shiny black seeds each with an orange aril which possibly attract birds that help in distributing seeds. It was recently reported that the main pigment in the bright orange aril of *Strelitzia*'s seed is the orange tetrapyrrole bilirubin found in animals which retains the colour years after the cell's death probably (Pirone et al., 2010).

At the juvenile stage, leaves are broad and oval resembling that of mature S. *reginae* but at a later stage the leaves grow smaller at the top and convert into a rush or upright needle-like feature (Winter, 2004; Sayers, 2007).

### In vivo plant propagation

### Vegetative

Vegetative multiplication is the natural asexual propagation of plants that results in newly produced plants having the identical genetic make-up of the mother plant. One of the most widely employed methods of vegetative propagation is the use of cuttings. This method makes use of vegetative parts such as stems, roots and leaves that have been removed from the mother plant. Vegetative propagation can also be done by the use of modified underground structures such as rhizomes, also known as suckers, as well as corms and tubers (Holley, 2009). *Strelitzia* species have rhizome structures which can be used for vegetative propagation (Burgess, 2004). Various authors reported on the vegetative propagation of S. *reginae* that is done either by separating the old plant into clumps or fans or by removing young offshoots from the plant (Hensley et al., 1998; Van der Walt, 2000; Burgess, 2004; Kantharaju et al., 2008). Division is limited by a low rate of multiplication of 0.5-1.5 divisions per branch per year, and the mother plant needs to be at least 10 years old before division (Van de Pol and Van Hell, 1988; Kantharaju et al., 2008). After dividing the old plant, the new clumps or divisions will require at least three months to generate new roots (Hensley et al., 1998). The new plants will require about two to three years to re-establish and to start flowering again (Van der Walt, 2000; Notten, 2002; Burgess, 2004). *Strelitzia juncea* has a much slower growth rate than other *Strelitzia* species. It does not multiply by suckers (offshoots) from the base of the stem but instead subdivision occurs between the middle leaves of each fan. The divided plants need about a year to re-establish and three to four years to flower (Winter, 2004).

Van de Pol and Van Hell (1988) reported that branching in S. *reginae* starts in the region of the apical dome with an absolute absence of branching from axillary buds. The absence of branching from the axillary buds may be due to a strong apical dominance effect (North et al., 2010). Mechanical induction of lateral branches in S. *reginae* was achieved by eliminating the apical dominance. Mature plants were divided into fans and the apex was removed by making a transverse incision above the basal plate throughout the basal leaf sheaths but still leaving the leaves in contact with the roots. After two to six months lateral sprouts (2-30 per fan) developed. Old roots were removed and after another six months new roots developed on the newly formed shoots where after individual plants could be split. The total period for this *in vivo* procedure took one year (Van de Pol and Van Hell, 1988).

Although this method seems to be more applicable than the separation of fans, the slow growth and seasonal variability of S. *reginae* still poses a serious drawback for mass propagation *in vivo.* Therefore, there is a need to explore the options for *in vitro* culture of this plant as well as for S. *juncea* and to improve its multiplication rate for horticultural purposes.

### By seeds

Seeds are used for propagation of many plants especially the ones that are difficult to propagate vegetatively. However, seeds of some plant species do not germinate readily after dispersal even when external conditions such as temperature, water and oxygen availability are suitable, thus remaining in a dormant stage (DuPont, 2011). Prevention of germination may be caused by physical dormancy such as a hard or impermeable seed coat and internal dormancy which include an immature embryo and the presence of an inhibitor or hormone such as abscisic acid (Yang et al., 2007; DuPont, 2011). Propagation of *Strelitzia* species by seeds is also a slow process. Fresh seeds of which the orange aril has been removed must be used and preferably sown in spring or early summer. Seeds kept under suitable conditions will germinate in six to twelve weeks (Hensley et al., 1998; Notten, 2002; Burgess, 2004). Seedling establishment can take about six months, producing one plant per seed (Sayers, 2007).The mature plants will only start flowering from four to seven years onwards (Kantharaju et al., 2008).

### Factors affecting seed germination

### Water and oxygen

Germination begins when dry seeds are hydrated with water and followed by embryo expansion (Finch-Savage and Leubner-Metzger, 2006). Seeds of S. *juncea* were found to have increased activity of glycolytic enzymes when treated with oxygen, indicating oxygen as a crucial requirement for their seed germination (De Meillon et al., 2002).

### Seed coat

The seed coat in dormant seeds possesses a physical barrier that prevents water and oxygen permeability (Yang et al., 2007). Germination in such seeds can be induced by scarification which weakens the seed coat thereby allowing more water and oxygen to diffuse into the seed and promote germination. In nature, seeds are subjected to mechanical scarification by birds or chemical scarification by passing through digestive tracts of animals which promotes seed germination when seeds are released into the environment (Baes et al., 2001). Artificial scarification can be achieved mechanically, chemically or by imbibition in hot water (Preece and Read, 2005; Shanmugavalli et al., 2007; DuPont, 2011).

Mechanical scarification requires that seeds be filed or nicked by hand using knives, or sand paper to remove the seed coat. The procedure works best for larger seeds since small seeds are difficult to handle. Pogroszewska (2006) reported that mechanical scarification promoted germination of S. *reginae* seeds by three fold while Hensley et al. (1998) and Burgess (2004) also advised that the outside of the seed must be scraped with a file to break the seed coat before sowing the seeds. The seeds will then germinate within 8-12 weeks if kept moist. Although the method may be effective for seeds of certain plant species, it is disadvantageous as it is laborious, time consuming and may damage the embryonic axis (Preece and Read, 2005).

Chemical scarification involves the use of chemical agents such as sulphuric acid, nitric acid and hydrochloric acid to weaken the seed coat (Jatt et al., 2007; Yang et al., 2007; DuPont, 2011). This is one of the most frequently used methods for breaking dormancy in seeds of many plant species with hard seed coats. Seeds of *Strelitzia* species are characterised by a hard seed coat and strong seed dormancy which makes it difficult to germinate (Barbosa et al., 2005). Both Hensley et al. (1998) and Winter (2004) recommended the soaking of seeds in concentrated sulphuric acid for five minutes to weaken the seed coat. Singh (2006) reported that soaking seeds of S. *reginae* and S. *juncea* in concentrated sulphuric acid for five minutes resulted in germination percentages of 62.5% in S. *reginae* and 25% in S. *juncea.* Barbosa et al. (2005) reported that the best results for seed germination and seed vigour of S. *reginae* were obtained for seeds that were soaked in concentrated sulphuric acid for nine minutes. Preliminary studies (unpublished data) by the Applicant showed that scarification of *Strelitzia* seeds in concentrated sulphuric acid can result in up to 70-80% germination *in vivo.* To date, the Applicant is not aware of studies which have been conducted on the *in vitro* seed germination of S. *juncea.* It is therefore important to evaluate the effect of scarification on seed germination of S. *juncea in vitro* under aseptic conditions.

### Plant growth regulators

Internal dormancy is caused by physiological conditions which delay germination and it is affected by internal factors such as an immature embryo and the presence of a plant growth hormone, notably abscisic acid (ABA) (DuPont, 2011). Gibberellic acid (GA₃) is a plant growth hormone that releases seed dormancy and its action is generally considered as antagonistic to ABA. Commercially, GA₃ is applied exogenously to release seed dormancy of many plant seeds.

Pogroszewska (2006) reported that 48 hours incubation of mechanically scarified seeds of S. *reginae* in a solution of GA₃ (800 mg/l) resulted in the greatest number of germinated seeds while seedling elongation was also stimulated. Singh (2006) also reported that treatment of S. *reginae* seeds with a solution of GA₃ at a concentration of 500 ppm for 48 hours alone and in conjunction with warm water at 50-55°C for half an hour improved seed germination. *Strelitzia reginae* seeds can be soaked in a solution of ethrel (2000 ppm) for 48 hours. This will lead to 80% seed germination within the first four to eight weeks. Without ethrel treatment approximately 50% of the seeds should germinate in four to eight weeks with a further 30% germinating sporadically for up to one year after sowing (Notten, 2002; Singh, 2006). Ethepon, an ethylene releasing compound is also used commercially for breaking seed dormancy. Winter (2004) indicated that scarifying seeds of S. *juncea* for five minutes in sulphuric acid and soaking them in a solution of ethephon (2000 ppm) or in GA₃ (800 mg/dm³) can improve seed germination.

### Temperature

Seeds require a particular temperature to initiate germination and temperature regimes vary from species to species. Seeds of some plant species require low temperatures to break seed dormancy while others need high temperatures. A constant temperature of 25°C is reported as most suitable for *in vivo* seed germination of S. *reginae* and S. *juncea* as low temperatures will retard or slow down germination (Notten, 2002; Winter, 2004). Seeds of S. *reginae* reached up to 30% germination at 25°C as compared to 15% germination at 30°C and 11% germination at 20°C (Barbosa et al., 2005).

### Light

Light environment plays an important role in germination of photosensitive seeds. The quality of light that reaches seeds is a characteristic of the light environment that affects seed germination. Seeds of many plant species from different environments respond to light through phytochrome, a light receptor pigment that is reversibly interconverted by red (660 nm) and far-red (730 nm) light between an inactive form (Pr) and a physiologically active form (Pfr) (Casal et al., 1998).

Seed germination of plant species with bigger seeds does not require light to germinate and such seeds, known as negatively photoblastic, can only germinate under dark conditions or when buried in soil. (Taiz and Zeiger, 2002). Limited reports are available on the effect of light quality on seed germination of *Strelitzia* species. Seeds of *Strelitzia* species have big seeds that germinate preferably when buried in soil at a depth of 1.5 times the size of the seeds (Winter, 2004). Singh (2006) reported that light assists germination in S. *reginae* seeds when the temperature ranges from 21-24°C. The applicant's preliminary studies (unpublished data) showed that seeds of S. *juncea* showed higher germination (70%) *in vivo* when exposed to total darkness as compared to red and white light treatments.

### In vitro plant propagation

### Factors effecting in vitro plant propagation

### Explant source

*In vitro* plant cultures are generally initiated from an explant such as meristematic cells, tissues and organs such as leaves, roots and other cell types like the embryo. The choice of explant is largely dependent on the type of culture (Pati et al., 2005). Explants can be obtained from plants grown in their natural environments or from plants grown under controlled conditions (Emek and Erdag, 2007). Explants obtained from plants grown under controlled conditions are desirable as they contain less or no contaminants compared to explants from plants grown in their natural environment (Pati et al., 2005).

Terminal and axillary meristems of mature S. *reginae* plants were previously used for initiation of *in vitro* cultures with partial or no success because of oxidative browning (North et al., 2010). Kantharaju et al. (2008) used a shoot tip explant for initiation of *in vitro* cultures of S. *reginae,* however, multiple shoot induction was unsuccessful due to oxidative browning. Micropropagation of S *reginae* using terminal buds of rhizomes was reported by Shreevatsa et al. (2004). This method is not considered suitable for culture of rare plants and limited plant material since plants are destroyed when their terminal and axillary buds are excised North et al. (2010).

Seeds can also be used for initiation of *in vitro* cultures. Zygotic culture is a nondestructive method and it offers many advantages as it results in high germination rate, bypassing seed dormancy, faster growth, limited wounding of explants and subsequently reduced browning of the media (North et al., 2010). In monocotyledonous plants, and especially in woody species such as conifers, propagation is only possible when zygotic embryos are used as explant source instead of older vegetative tissues (Bonga et al., 2010). Paiva et al. (2004) indicated the use of isolated embryos as explants for *in vitro* propagation of S. *reginae* while Fernandez et al. (2008) used cotelydon node fragments from 30 days old plantlets to induce somatic proembryos. These proembryos produced a root meristem-like organisation but could not develop a shoot meristem and further development of proembryos was prevented (Fernandez et al., 2008). North et al. (2010) presented a detailed review on possible ways for *in vitro* propagation of *Strelitzia* species using embryos as the starting material. The review discussed the possible effects of various factors such as media compositions on *in vitro* germination of immature embryos, plant growth regulators and suppressing of apical dominance to induce multiple shoots from axillary buds, and the use of antioxidants and adsorbents to reduce oxidative browning of wounded tissues. Further studies by North et al. (2011) indicated the successful *in vitro* germination of immature embryos of S. *reginae.* However, no multiple shoot induction was reported. To date, no reports are available for any *Strelitzia* species on *in vitro* propagation using seeds as starting material with the attempt to induce multiple shoots from *in vitro* produced seedlings.

### Aseptic conditions

Generally, *in vitro* plant cultures are conducted aseptically in a laminar flow cabinet and kept under aseptic conditions to avoid microbial contaminations (Steward, 2008). Explants are surface sterilised with various sterilants such as ethanol, sodium hypochlorite (NaOCl), calcium chlorite and mercury chloride (Badoni and Chauhan, 2010; Vasudevan and Van Staden, 2010). Sterilisation of seeds requires a longer exposure to sterilants because they have much thick tissues compared to soft tissues in leaves. For establishment of zygotic cultures, S. *reginae* seeds were surface-sterilised with 70% ethanol for 30 seconds followed by 1.5% solution of sodium hypochlorite (NaOCl) with two drops of Tween-20 for 15 minutes and then rinsed several times with sterile distilled water (North et al., 2011).

### Culture media

Various plant tissue culture media differing in their inorganic, organic and hormone compositions have been formulated. Essential macronutrients (nitrogen, phosphorus, magnesium, calcium, sulphur and potassium), micronutrients (iron, manganese, copper, zinc, molybdenum, boron and cobalt) and vitamins (thiamine, pyridoxine and nicotinic acid) are the main components of the basal media and can vary from culture to culture (Murashige and Skoog, 1962; Pati et al., 2005). The most commonly used basal culture media is the Murashige and Skoog (MS) media which was developed for tobacco plants (Murashige and Skoog, 1962; Pati et al., 2005). Sucrose is the most commonly used carbon source but may vary in concentration depending on the explant source and type of culture. Gelling agents such as agar, Phytagel and Gelrite are commonly used to provide support for the explants (Pati et al., 2005; Ozel et al., 2008). Agar is frequently used because of its thermo-stability (Pati et al., 2005), however, Phytagel and Gelrite are increasingly used due to their clarity and purity (Aasim et al., 2009).

Explants release polyphenolic compounds into the media which are oxidised and cause browning of the media (Pan and Van Staden, 1998). Failure to establish tissue culture techniques for *in vitro* propagation of S. *reginae* due to oxidative browning of explants was reported earlier (North et al., 2010). Oxidative browning occurs due to wounding of tissues which release polyphenolic compounds that diffuse into the media (Madhusudhanan and Rahiman, 2000; Kantharaju et al., 2008). These exudates were found to be detrimental to the growth of shoot tip explants of S. *reginae* as they caused the onset of necrosis (Kantharaju et al., 2008). The oxidative browning of the explants has been reported to be controlled by the use of antioxidants such as ascorbic acid (AA), citric acid, polyvinylpyrollidone (PVP) (Madhusudhanan and Rahiman, 2000; Kantharaju et al., 2008) and the use of adsorbent activated charcoal (AC) (Pan and Van Staden, 1998; Madhusudhanan and Rahiman, 2000; North et al., 2011). Although the antioxidant treatment showed to promote the growth of embryos isolated from seeds of *S*. *reginae,* browning was still a problem (Paiva et al., 2004). North et al. (2011) reported on the effects of various media compositions and the addition of activated charcoal and vitamins on the *in vitro* germination and discoloration of S. *reginae.*

### Plant growth regulators

*In vitro* growth responses of plant tissues are largely dependent on exogenous application of plant growth regulators such as auxins, cytokinins and gibberellins. The response however, depends on the hormone combination, concentration and the type of explants. Cytokinins are responsible for cell division and induction of adventitious or axillary shoots while auxins promote adventitious root formation.

Limited reports are available on the effect of plant growth regulators on multiple shoot induction of *Strelitzia* species (Shreevatsa et al., 2004). Kantharaju et al (2008) cultured shoot tip explants of S. *reginae* on MS media supplemented with 5 mg/l BAP, however no multiple shoot induction was reported.

### Growth conditions

*In vitro* plant cultures require specific temperature, light intensity and quality for proper growth. Plant tissue cultures are usually kept in growth rooms under controlled light and temperature conditions. For most plant tissue cultures, temperature ranges from 22-25°C (Pati et al., 2005). A photoperiod of 14-16 hours) and light intensity of 25-50 µmol m⁻²s⁻¹ supplied by cool white influorescence lamps are usually provided for optimal growth of shoot induction cultures (Pati et al., 2005). Shoot induction and embryo cultures of S. *reginae* were also reported to be maintained at 25°C under light intensity of 50 µmol m⁻²s⁻¹ (Kantharaju et al., 2008; North et al., 2011).

### Acclimatisation

Generally, *in vitro* regenerated plants are removed from their culture media, transplanted into a mixture of sand, soil, vermiculite and compost and gradually exposed to low humidity for acclimatisation to natural environment. This is a crucial stage of plant tissue culture when plants are produced for commercial purposes. Having considered the information above, it is apparent that all *in vitro* tissue culture studies of *Strelitzia* species, such as S. *reginae,* up to date, have resulted in only partial success and low multiplication rate, indicating major problems with *in vitro* propagation and multiplication of this recalcitrant plant species (North et al., 2010). However, no plant tissue culture studies have been reported on *S. juncea.* Therefore, it is important to evaluate and optimise the conditions for micro-propagation of this economically valuable plant species with high horticultural potential.

### SUMMARY OF INVENTION

Aspects of the present invention are set out in the independent claims. Preferred embodiments of these aspects are set out in the dependent claims.

The invention aims to provide a commercially viable method for rapid *in vitro* mass propagation of *Strelitzia juncea* and other *Strelitzia* species using *in vitro* produced seedlings or shoots as an explant source for multiple shoot induction and plant regeneration.

In accordance with a first aspect of the invention, there is provided an *in vitro* method for the propagation of a *Strelitzia* species from a *Strelitzia* seed, the method including the steps of sequentially:
(a) chemically scarifying the *Strelitzia* seed;
(b) thereafter, effecting surface sterilisation of the scarified seed;
(c) optionally, imbibing the scarified and sterilised seed in an anti-oxidant solution;
(d) inoculating the scarified and sterilised seed on germination media and allowing the seed to germinate in darkness to establish a germinated seed culture;
(e) in a seedling development step, subjecting the germinated seed culture to light conditions for a suitable photoperiod for seedling development;
(f) obtaining a shoot explant by removing the root of the seedling;
(g) mechanically removing an apical dome of the shoot explants while retaining a leaf sheath of the shoot explants, thereby uplifting apical dominance in the shoot explant;
(h) culturing the shoot explants with the removed apical dome on basal culture media containing a plant growth regulator, thereby initiating multiple shoot development on the shoot explant with the removed apical dome, or when the germination media comprises basal culture media supplemented with a plant growth regulator, culturing the scarified and sterilised seed directly, thereby initiating multiple shoot development;
(i) separating the resulting multiple shoots and, if required, subculturing axillary shoots to shoot induction media for further multiplication;
(j) transferring axillary shoots to root development media for inducing root growth, and hence plantlet regeneration;
(k) acclimatising the resultant *in vitro* produced plantlet.

It will be appreciated that, normally, the method will involve applying its steps simultaneously to a plurality of seeds so that a number of seedlings, shoots, plantlets, etc will be produced. The resulting seedlings, shoots and plantlets are suitable for use as explants.

The *Strelitzia* species may be *Strelitzia reginae* or *Strelitzia juncea.* More particularly, the *Strelitzia* species may be *Strelitzia juncea.*

An orange aril on the seed has been shown to inhibit germination of the seed. Accordingly, the method of the invention may include a step of mechanically removing the aril from the seed material.

The chemical scarification of the seed material may be carried out by incubation of the seed in concentrated sulphuric acid. More particularly, the chemical scarification of the seeds may be carried out by incubation of the seed in 99% sulphuric acid. A person skilled in the art of this invention may be aware of other chemicals which may have the effect of breaking the dormancy of the seeds. The person skilled in the art of this invention may also be aware of mechanical means of seed scarification.

The seed germination media in accordance with the invention may be, but not limited to, a full strength MS basal media with or without PGRs, and supplemented with vitamins, reduced level of sucrose and gelling agent. A person skilled in the art of the invention will be aware of the MS media (Murashige and Skoog, 1962).

The position of the hilum of the seed has been demonstrated to have an effect on germination thereof. According to this invention, the step of inoculating the seed on the seed germination media may include placing the seed on the media, with the hilum of the seed positioned above the media.

When the imbibing of the scarified and sterilised seed in the anti-oxidant solution is effected, the imbibed seed displays a germination rate similar to unimbibed seed or better, depending on the freshness of the seeds, but produces a stronger seedling that is more suitable as an explant source for axillary shoot induction. The anti-oxidant may be ascorbic acid. The anti-oxidant solution may be an aqueous solution containing about 100-200 mg/l ascorbic acid. More particularly the anti-oxidant solution may be a solution containing about 150 mg/l ascorbic acid. The period of imbibition may be from 12-24 hours.

The seed may be allowed to germinate for 2-3 weeks.

The seedling development step may include transferring the germinated seed culture to the light conditions. It may also include development of the seedlings under controlled conditions. The controlled conditions may include temperature at 25°C and light intensity of about 50-90 µmol m⁻²s⁻¹. The controlled conditions will also include the photoperiod which may be from 12-16 hours, e.g. 14-16 hours. The seedling development step may be carried out over about 4-9 weeks.

The seedlings produced by the above method may remain healthy and viable in the seed germination culture for up to about six (6) months without sub-culturing.

The step of obtaining of shoot explants from *in vitro* produced seedlings may be carried out by mechanical removal of the root below the basal plate. The removal of the root may be performed under an anti-oxidant solution, thereby avoiding the oxidative browning of the tissue.

The step of uplifting the apical dominance of a shoot explant is thus carried out by mechanical removal of the apical dome of the shoot explant while retaining a leaf sheath of the shoot explant. The mechanical removal of the apical dome may be carried out by making a nick above the basal plate of the shoot explant. The removal of the apical dome may be performed under an anti-oxidant solution, thereby avoiding the oxidative browning of the tissue.

A person skilled in the art of the invention will be aware of several anti-oxidants which may be used for this purpose. The anti-oxidant solution may be an ascorbic acid solution. The anti-oxidant solution may also be an ascorbic acid solution in combination with polyvinylpyrrolidone (PVP).

The multiple shoot induction step may be initiated by culturing shoot explants with a removed apical dome on MS shoot-induction media containing full strength sucrose and a plant growth regulator(s). The plant growth regulator may be 6-benzylaminopurine (BAP) alone or in combination with indole-3-butyric acid (IBA) and/or α-naphthaleneacetic acid (NAA).

The multiple shoot induction step may also be initiated directly from seeds by culturing scarified and sterilised seeds on MS media containing half strength sucrose and a plant growth regulator(s). The plant growth regulator may be 6-benzylaminopurine (BAP) alone or in combination with indole-3-butyric acid (IBA) and/or α-naphthaleneacetic acid (NAA).

The multiple shoot induction step may be further initiated by subculturing the *in vitro* induced axillary shoots on MS shoot-induction media containing full strength sucrose and a plant growth regulator(s). The plant growth regulator may be 6-benzylaminopurine (BAP) alone or in combination with indole-3-butyric acid (IBA) and/or α-naphthaleneacetic acid (NAA).

The step of elongation of the induced axillary buds/shoots (1-2 cm length), may be carried out by sub-culturing into PGR-free MS basal media containing full strength sucrose and gelling agent.

The step of root development may be carried on axillary shoots derived from the multiple shoot development step. The development of roots from the axillary shoots is induced by transferring the shoots on solid or liquid rooting culture media. The solid rooting culture media may contain MS basal media supplemented with vitamins, full strength sucrose (30 g/l) and 2-8 g/l solidifying agent. The liquid rooting culture media may comprise of MS basal media supplemented with vitamins and without sucrose and solidifying agents. The solid and the liquid culture media, both may contain root inducing plant growth regulators. A person skilled in the art of this invention may be aware of several root inducing growth regulators. The root development inducing PGRs may include, but not limited to, 1-5 mg/l IBA or NAA. Accordingly the MS basal media may comprise 1-2 mg/l IBA, 2-3 g/l Gelrite and 30 g/l sucrose.

The step of inducing shoot development and the step of inducing root development may be conducted simultaneously in shoot explants with a removed apical dome. The simultaneous (in the same culture media) development of the axillary shoot and the adventitious root may be induced by culturing the explants in media containing combinations of shoot inducing PGRs (BAP) and root inducing PGRs (NAA).

According to a second aspect of the invention, there is provided an *in vitro* method for rendering a *Strelitzia* seed more amenable to germination, the method including the steps of sequentially:
(a) chemically scarifying the *Strelitzia* seed;
(b) thereafter, effecting surface sterilisation of the scarified seed; and
(c) imbibing the scarified and sterilised seed in an anti-oxidant solution; and
(d) placing the scarified and sterilised seed on seed germination media, with a hilum of the seed positioned above the media and allowing the seed to germinate.

The chemical scarification, surface sterilisation and imbibition may be as herein before described in respect of the first aspect of the invention.

The invention will now be described in more details with reference to the following non-limiting examples and accompanying figures.

### BRIEF DESCRIPTION OF THE DRAWINGS

In the figures:

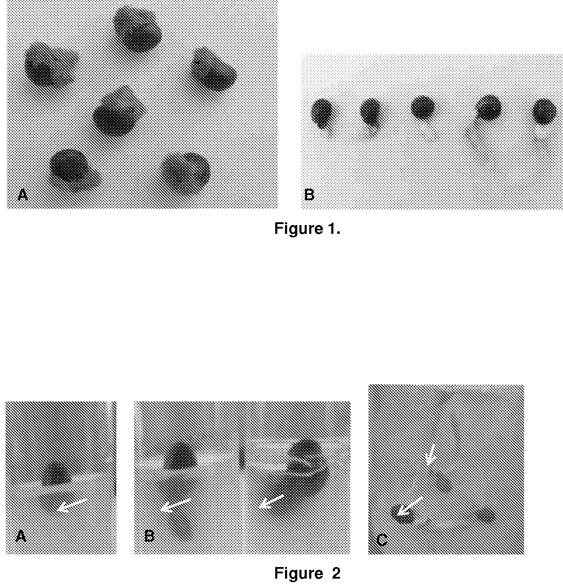

FIGURE 1 shows, for Example 1, the non-scarified seeds of *S. juncea* with orange aril (A) and germinated, scarified, seeds without an aril (B);
FIGURE 2 shows, for Example 1, the effect of scarification and seed position on *in vitro* seed germination and seedlings development of *Strelitzia.* (A) No germination of non-scarified seed, (B) germination of scarified seeds without seedling development if seeds are positioned vertically, with the hilum embedded, into the media. (C) Seed germination and seedling development of scarified seeds if the hilum is above the culture medium;

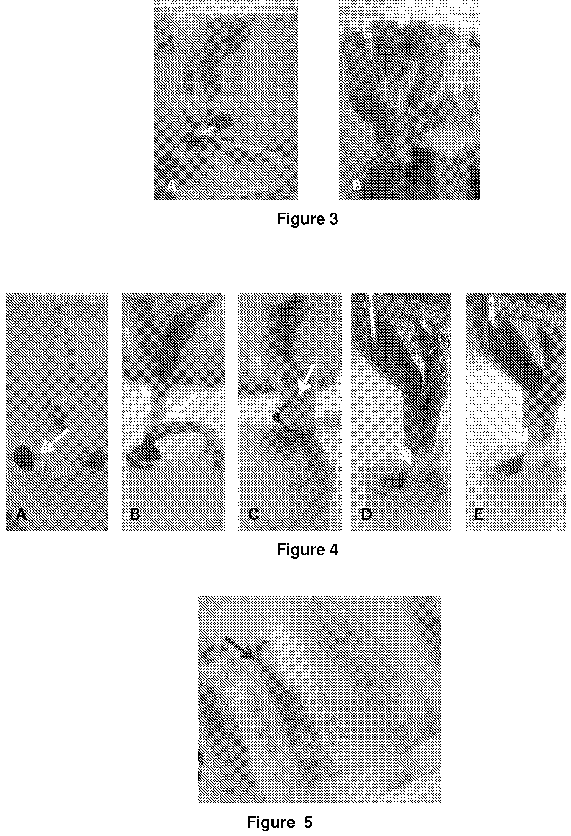

FIGURE 3 shows, for Example 1, seedlings produced from *in vitro* germinated seeds. (A) One month old seedling; (B) 4-5 month old seedling;
FIGURE 4 shows, for Examples 1 and 2, seedlings developed *in vitro* from non-imbibed seeds (A), seeds imbibed, prior to germination in: distilled water for 12 hours (B), and in ascorbic acid for 12 hours (C), 18 hours (D) and 24 hours (E);
FIGURE 5 shows, for Example 1 and 2, the germination of seeds and seedling development of *Strelitzia* under aseptic conditions in plastic culture vessels;

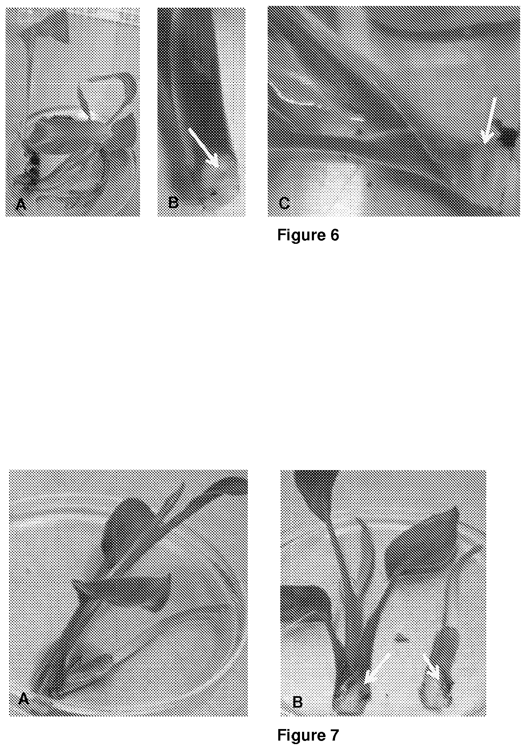

FIGURE 6 shows, for Example 2, the examples of explant preparation from *in vitro* produced seedling with roots (A); Removal of the seedling's roots (below the basal plate) (B) and the apical dome (V-shape cut above the basal plate) (C) to obtain shoot explants for axillary shoot induction ;
FIGURE 7 shows, for Example 3, explant preparation from *in vitro* multiplied primary axillary shoots (A); Axillary shoot explant with the apical dome removed (B);

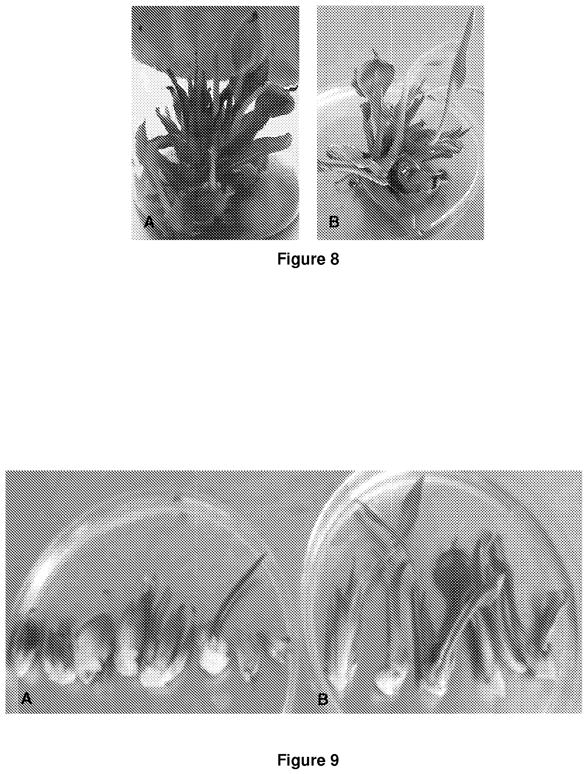

FIGURE 8 shows, for Examples 2 and 3, the proliferation of secondary multiple axillary shoots on shoot explant cultured on shoot induction media comprising of a MS basal culture medium, 2.0 mg/l BAP and 1.0 mg/l IBA, and 2-3 g/l Gelrite, and 30 g/l sucrose for 12-14 (A and B respectively) weeks;
FIGURE 9 shows, for Examples 2 and 3, splitting of secondary axillary shoots obtained from *in vitro* culture of primary axillary shoot explant. (A) Small axillary shoots (less than 3 cm in length) that can be sub-cultured for elongation on a PGR-free MS culture medim; (B) Large axillary shoot explants that can be transferred to rooting media;

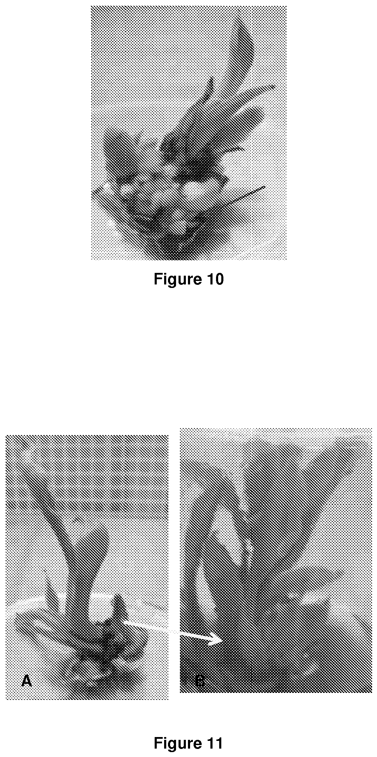

FIGURE 10 shows, for Examples 2 and 3, the proliferation of numerous, third generation, of axillary buds/shoots on secondary axillary shoot explant obtained from previous cultures (Figure 9 B) when-sub cultured *in vitro* on media formulated in the invention (MS basal culture medium, 2.0 mg/l BAP and 1.0 mg/l IBA, 2-3 g/l Gelrite and 30 g/l sucrose) for 6-8 weeks;
FIGURE 11 shows, for Example 3, the elongation of axillary buds/shoots after subculture on PGR-free media comprising of MS basal culture medium, 2-3 g/l Gelrite and 30 g/l sucrose. (A) Buds/shoots prior elongation; (B) Elongated axillary shoots after 6-8 weeks in culture;

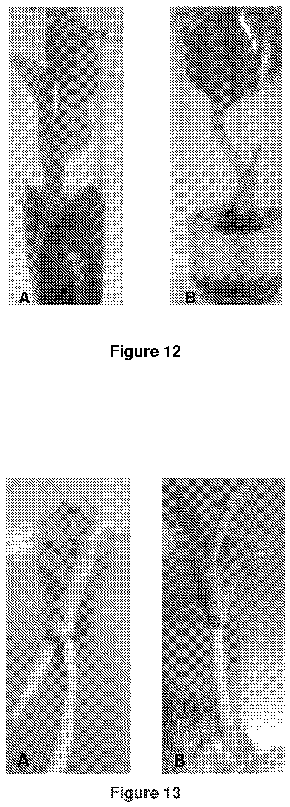

FIGURE 12 shows, for Example 4, the root induction at the base of, *in vitro* obtained, axillary shoots on media comprising of MS basal culture medium, 1 mg/l IBA, 2-3 g/l Gelrite, and 30 g/l sucrose and activated charcoal (A). Absence of root formation if the shoot explant is cut few mm below the basal plate (B);
FIGURE 13 shows, for Example 4, the examples of a *Strelitzia* plantlets regenerated *in vitro* using the procedures developed in this invention;

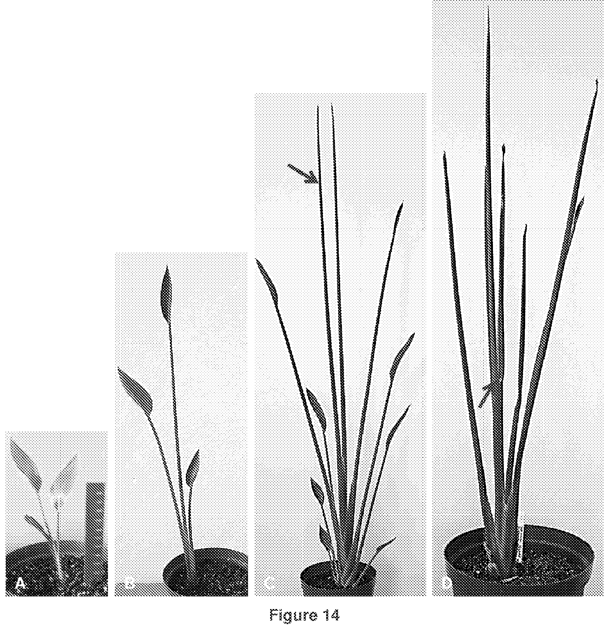

FIGURE 14 shows, for Example 4, the examples of acclimatised *Strelitzia* plants regenerated *in vitro* according to this invention, after the transplanting to soil comprising of a 2:1:1 mixture of potting soil, vermiculite and sand. Plants kept under controlled growth conditions at 25°C, photoperiod of 14-16 hours and light intensity of 150-200 µmol m⁻²s⁻¹ for: (A) 3-4 months, juvenile stage with broad leaves, (B) 12-13 months, ready to be transferred into gardens, (C) 24 months, new leaves grow smaller at the top, (D) 30 months, new leaves convert into a rush and subdivision between the middle leaves of the fan occurs, suitable for vegetative propagation.

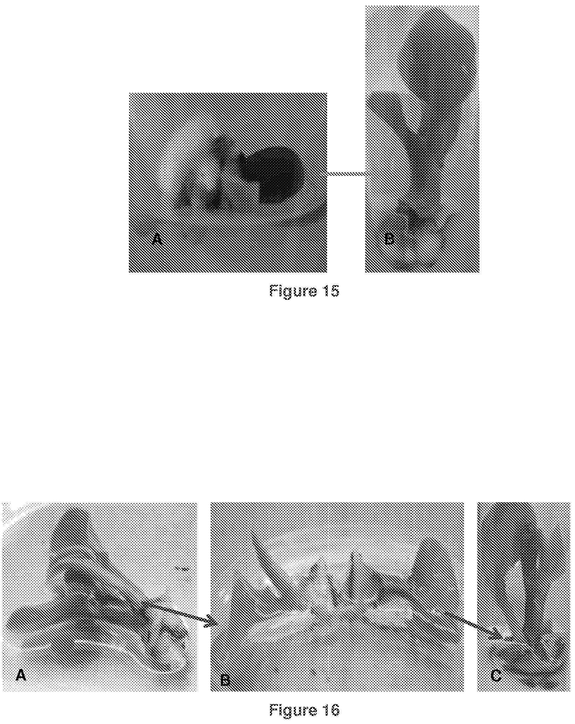

FIGURE 15 shows, for Example 5, germinated seed with hairy roots (A) and a seedling with multiple roots developed after 4-5 weeks of subculture on MS media containing 2 mg/l BAP and 1 mg/l NAA;
FIGURE 16 shows, for Example 5, the examples of *in vitro* induction of both primary axillary shoots and adventitious roots on *Strelitzia's* shoot explants when cultured on media comprising of MS basal culture medium, PGR (2 mg/l BAP, and 1mg/l NAA), 2-3 mg/l Gelrite, and 30 g/l sucrose (A). Separation of the primary axillary shoots and adventitious roots under antioxidant solution (B). Proliferation of new secondary axillary shoots and adventitious roots (C) on axillary shoot explant obtained from previous cultures (A and B), after subculture on the same media for 3-4 weeks;

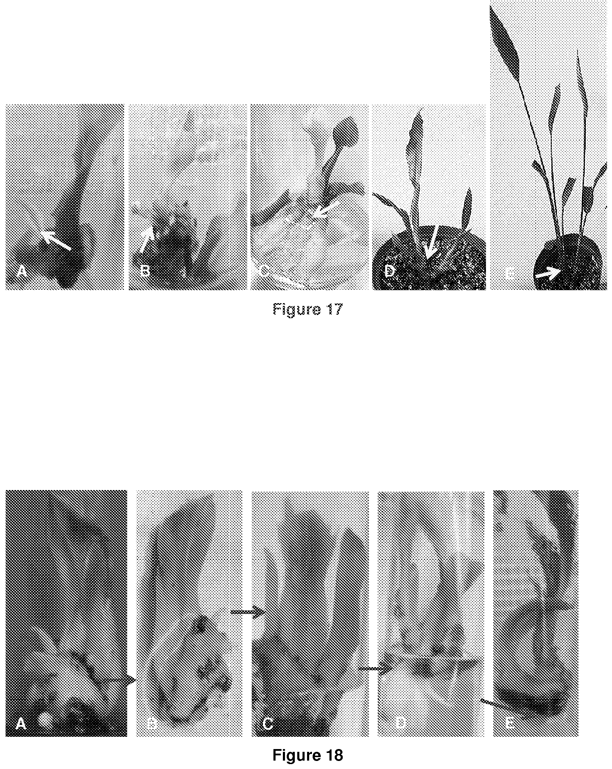

FIGURE 17 shows, for Example 5, the examples of *in vitro* induction of both axillary shoots (B) and adventitious roots (C) from *Strelitzia's* secondary axillary shoot explants (A) when cultured on media comprising of MS basal culture medium, PGR (2 mg/l BAP, and 1 mg/l NAA), and 2-3 g/l Gelrite, and 30 g/l sucrose; Examples of acclimatised *Strelitzia* plants regenerated *in vitro* according to this invention after the transfer to a soil mixture comprising of a 2:1:1 of potting soil, vermiculite and sand for: (D) 2-3 months and (E) 12-13 months;
FIGURE 18 shows, for Example 6, the examples of in *vitro* multiplication and regeneration of *Strelitzia* from a basal meristem of a shoot explant (apical dome removed). Induction of axillary buds after 8 weeks of culture on shoot induction media comprising of MS basal culture media, combination of three PGRs (2 mg/l BAP, 1 mg/l IBA, 1 mg/l NAA), 2-3 g/l Gelrite, and 30 g/l sucrose (A and B); Shoot elongation and new axillary shoot proliferation after one-two weeks of subculture on the same shoot induction media (C and D). Root induction on the base of axillary shoots after subculture to a rooting MS culture media supplemented with 1 mg/l IBA (E);

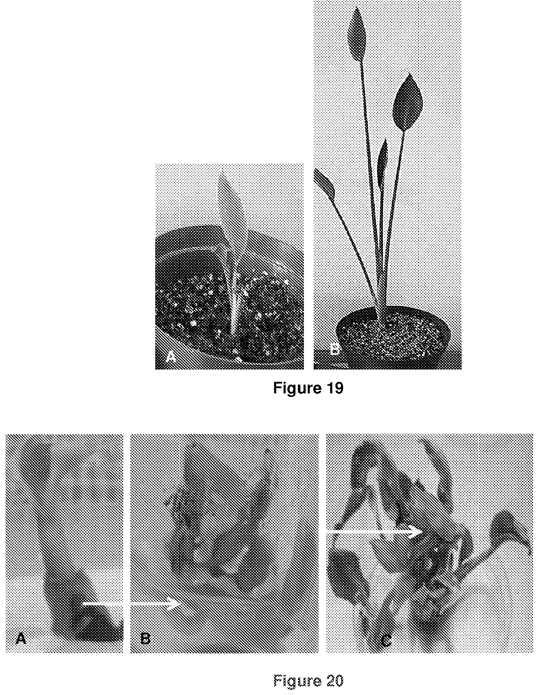

FIGURE 19 shows, for Example 6, example of acclimatised *Strelitzia* plants, with multiple shoots and roots, regenerated *in vitro* according to this invention, after the transfer to a soil mixture comprising of a 2:1:1 of potting soil, vermiculite and sand for (A) 3-4 months and (B) 12-13 months ready to be transferred into gardens;
FIGURE 20 shows, for Example 7, examples of *in vitro* induction of multiple (10-12) axillary buds and/or shoots (B) from *Strelitzia's* shoot explant (apical dome removed) (A) when cultured on media comprising of MS basal culture media, PGR (1 mg/l BAP), 2-3 g/l Gelrite, and 30 g/l sucrose. Elongated axillary shoots (C) after the subculture of multiple axillary bud/shoot cluster (B) to the same culture media for 4-6 weeks;

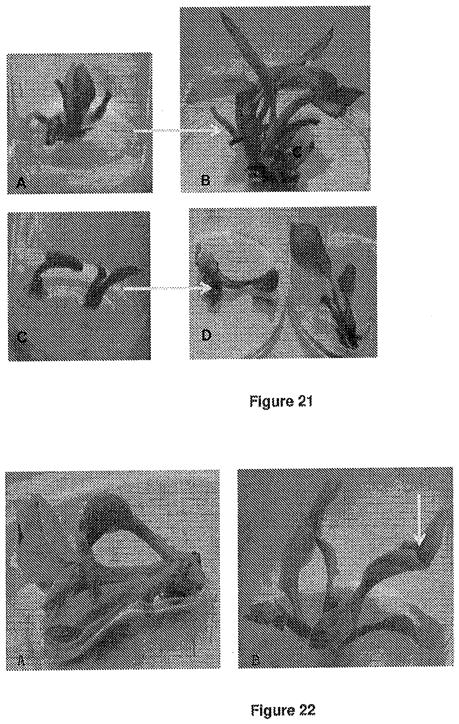

FIGURE 21 shows, for Example 7, examples of *in vitro* induction of new multiple, secondary, axillary buds and shoots (B and D) on primary axillary shoot explant from previous cultures (Figure 20A and C) after three weeks of subculture on shoot induction media, comprising of MS basal culture medium, PGR (1 mg/l BAP), 2-3 g/l Gelrite, and 30 g/l sucrose;
FIGURE 22 shows, for Example 7, examples of *in vitro* induction of (A) axillary shoots on shoot explant cultured on MS basal culture medium containing PGR cytokinin (2 mg/l BAP), 2-3 g/l Gelrite, and 30g/l sucrose and (B) elongated shoots with subdivision of the leaf after 3 weeks of subculture on the same media; and

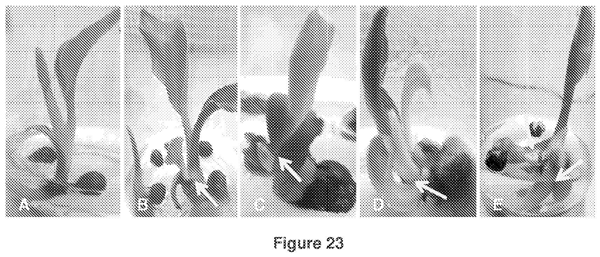

FIGURE 23 shows, for Example 8, (A) a seedling developed on PGR-free, half strength sucrose, MS seed germination media; Multiple shoot induction on seeds germinated *in vitro* on germination media comprising of MS basal culture medium, 2-3 mg/ml Gelrite, half strength sucrose (15 g/l) and PGRs such as: (B) 2 mg/l BAP; (C) 2mg/l BAP +1 mg/l IBA (B); (D) 2 mg/l BAP + 1 mg/l IBA +1 mg/l NAA and (E) BAP +1 mg/l NAA.

### DETAILED DESCRIPTION OF THE INVENTION

### Definitions:

"Aril" refers to an appendage growing at or near the hilum of a seed; fleshy thickening of the seed coat;
"Explant" refers to a plant organ or tissue, suitable for culture in an artificial nutrient media;
"Rapid" refers to a healthy, *Strelitzia,* plant with a shoot length of from about 5 cm to 10 cm suitable for transplanting to soil that may be produced *in vitro* from a basal axillary meristem for about 6 months or less;
"Induce" refers to stimulate or support of a growth response;
"Micropropagation" refers to *in vitro* regeneration of plants from organs, tissues, cells or protoplasts and the true-to-type propagation of a selected genotype using *in vitro* culture technique;
"MS" refers to Murashige and Skoog's culture media.

"PGR" refers to plant growth regulator(s) or (PGRs)
"BAP" refers to 6-benzylaminopurine
"IBA" refers to Indole-3-butyric acid
"NAA" refers to α-naphthaleneacetic acid
"Kin" refers to kinetin
"AA" refers to ascorbic acid
"PVP" refers to polyvinyl-pyrrolidone
"Primary shoot explant" refers to a shoot derived from *in vitro* produced, *Strelitzia,* seedling by removing the root of a seedling
"Primary axillary shoot" refers to the first *in vitro* produced culture of axillary shoots from a shoot explant
"Secondary axillary shoot" refers to the second *in vitro* produced culture of axillary shoots from a primary axillary shoot explant.

The following apply to all the Examples:

### Plant material

Seeds of *Strelitzia (juncea*) species were purchased from Silverhill Seeds nursery in Cape Town, and the study was conducted at the tissue culture laboratory of the Biodiversity Department at University of Limpopo. Seeds were used to produce seedlings which served as an explant source for obtaining basal meristematic tissues for *in vitro* multiplication.

### Micropropagation of Strelitzia (S. juncea)

"Micropropagation" can be performed using plant tissue standard culture methods. It typically involves four major stages (Hartmann et al., 2002) such as: 1) Establishment and stabilisation of explants in culture may include: choice of explant, explant disinfection, culture media preparation and sterilisation, explant stabilisation; 2) Multiplication may include: culturing of explants in shoot induction media to generate multiple shoots, sub-culturing of shoots to elongation media 3) Root formation may include: transferring the elongated shoots to rooting culture media *in vitro* or *ex vitro* in soilless cultures and high humidity. 4) Acclimatisation of the *in vitro* produced plantlets to outdoor environment.

### Aseptic conditions

All *in vitro* procedures were performed in a laminar flow cabinet following standard aseptic procedures as known in the field of plant tissue culture. All culture vessels, glassware and dissecting instruments were sterilised by autoclaving for 20 minutes at 120°C prior the experiment. Dissecting instruments were flame-sterilised during the inoculation procedures.

### Culture media

The basal media used to culture *Strelitzia* may be any of those already known in the field of plant tissue culture, such as Murashige and Skoog (MS), Gamborg's (B5), Woody Plant Medium (WPM) and a like. The preferred basal media used for the *in vitro* culture of *Strelitzia* was, but not limited to, full strength MS culture media. Basal media can also be supplemented with various carbon sources at various concentrations depending on the type of culture and the explant.

The carbon source of the culture may be sucrose or glucose, at a concentration of about 2-5%. The carbon source may also be sugar alcohol like myo-inositol, typically, at a concentration of about 1-5%. In some preferred embodiments in this invention, the basal media for seed germination may include sucrose at a concentration of about 0.75-1.5%. In other preferred embodiments, the basal media for shoot induction will include sucrose at concentration 3%.

In some embodiments, the basal media may include gelling agents such as agar and Gelrite at concentrations of about 0.1-1%.

The MS media was prepared using stock solutions or solids of the constituents as indicated in Table 1. Culture media for shoot and root induction was supplemented with various combinations and concentrations of PGR's (Table 2). The pH of the culture media was adjusted to 5.8 and the media was autoclaved for 20 minutes at 120°C.

**Table 1. Composition and preparation of MS media cited in Dixon and Gonzales (1995)**

| **Constituent** | **Molarity in medium** | **Concentration of stock solution (mg/l)** | **Volume of stock per litre of medium (ml)** |
|---|---|---|---|
| **Major inorganic nutrients** | | | |
| NH₄NO3 | 2.06 × 10⁻² | 33 000 | |
| KNO₃ | 1.88 × 10⁻² | 38 000 | 50 |
| CaCl₂.2H₂O | 3.00 × 10⁻³ | 8800 | |
| MgSO₄.7H₂O | 1.50 × 10⁻³ | 7400 | |
| KH₂PO₄ | 1.25 × 10⁻³ | 3400 | |

| **Trace elements** | | | |
|---|---|---|---|
| KI | 5.00 × 10⁻⁶ | 166 | 5 |
| H₃BO₃ | 1.00 × 10⁻⁴ | 1240 | |
| MnSO₄.4H₂O | 9.99 × 10⁻⁵ | 4460 | |
| ZnSO₄.7H₂O | 2.00 × 10⁻⁵ | 1720 | 5 |
| Na₂MoO₄.2H₂O | 1.00 × 10⁻⁶ | 50 | |
| CuSO₄.5H₂O | 1.00 × 10⁻⁷ | 5 | |
| CoCl₂.6H₂O | 1.00 × 10⁻⁷ | 5 | |

| **Iron source** | | | |
|---|---|---|---|
| FeSO₄.7H₂O | 1.00 × 10⁻⁴ | 5560 | 5 |
| Na₂EDTA.2H₂O | 1.00 × 10⁻⁴ | 7460 | |

| **Vitamins** | | | |
|---|---|---|---|
| Myo-Inositol | 4.90 × 10⁻⁴ | 20 000 | |
| Nicotinic acid | 4.66 × 10⁻⁶ | 100 | |
| | | | 5 |
| Pyridoxine-HCL | 2.40 × 10⁻⁶ | 100 | 5 |
| Thiamine-HCL | 3.00 × 10⁻⁷ | 100 | |
| Glycine | 3.00 × 10⁻⁵ | 400 | |

| **Carbon source** | | | |
|---|---|---|---|
| Sucrose | 8.80 × 10⁻² | | 15-30 g/l (added as solid) |

| **Solidifying agents** | | | |
|---|---|---|---|
| Agar | 8.80 × 10⁻² | | 8 g/l (added as solid) |
| Gelrite | | | 2-3 g/l (added as solid) |

### Plant growth regulators culture media

The present invention provides for a method where explants with basal meristem are grown in media containing selected concentrations and combinations of PGRs. The PGRs used in the media can be any phytohormone that may affect growth in the desired manner during different stages of tissue culture. Examples of suitable PGRs for this invention include natural or synthetic auxins and cytokinins.

The cytokinins used for *in vitro* culture of *Strelitzia* can include, but are not limited to, 6-benzylaminopurine (BAP), and kinetin (Kin). The auxins used can include, but are not limited to, naphthaleneacetic acid (NAA), and indole-3-butyric acid (IBA), or the like and their derivatives.

The PGR may be used singly or in combination with two or more other phyto-hormones. The concentration of the PGR present in the media can be varied depending on the explant and type of culture. The present invention provides that concentration of PGR is between 1-5 mg/l. In preferred embodiments, media for shoot induction may contain 1-2 mg/l of cytokinin singly or in combination with 1 mg/l of auxins (Table 2). In the preferred embodiments for root induction, 1-2 mg/l of auxins in the media may be used.

### Seed culture

### Seed germination

Scarified and sterilised seeds were cultured on hormone free MS culture media supplemented with vitamins, half strength concentration (15 g/l) of sucrose and 2-3 g/l Gelrite. In some embodiments seeds were imbibed (soaked) for different periods of time (6-24 hours) in AA (100-200 mg/l or distilled water prior culturing in culture media. In other embodiments the seed germination media was supplemented with PGRs such as 6-benzylaminopurine (BAP) alone or in combinations of indole-3-butyric acid (IBA) and α-naphthaleneacetic acid (NAA) (Table 2).

In some embodiments, seeds were placed vertically and half of the seed, with the hilum, were embedded into the media, while the other half of the seed remained above the culture media.

In the most preferred embodiment, the seeds were placed with the hilum on the surface of the media to ensure better aeration since germination of *Strelitzia* seeds is oxygen sensitive.

Germination for all seed cultures was regarded as the emergence of the radicle from the seed and it was observed on a weekly basis.

### Seedling development

In most preferred embodiment for seedling establishment, cultures with germinated seeds were transferred from darkness to light conditions, with 14-16 hours photoperiod and light intensity of 50-90 µmol m⁻²s⁻¹. Seedlings developed in these cultures after 4-5 weeks may serve as an explant source for multiple shoot induction. The *in vitro* produced seedlings can remain healthy and viable in the germination culture, without the need of sub-culturing, up to 4-5 months. This procedure has a potential for commercial application since it is cost- and time- efficient, and may ensure high germination percentage of expensive *Strelitzia* seeds in a short period of time, thus providing a healthy stock for a long period of time.

In another embodiment, scarified and sterilised seeds were germinated and aseptic seedlings were produced in sterile plastic culture vessels lined with sterile wet tissue under light conditions with 14-16 hours photoperiod and light intensity of 50-90 µmol m⁻²s⁻¹. This procedure is more cost- and time- effective (no culture media is required) for commercial seedling production as compared to the seedlings produced on MS culture media and may be suitable for mass propagation of *Strelitzia.*

To the Applicant's knowledge, there are no reports on successful seed germination and seedling production of *Strelitzia in vitro* or under aseptic conditions. Some studies have used isolated embryos for *in vitro* culture with low or no success due to oxidative browning. The methods used in this invention provide commercially viable procedures for rapid and high success rate of seed germination and seedling development which also can serve as an explant source for *in vitro* multiplication. This invention could be beneficial for large scale production of *Strelitzia* spp. and S. *juncea* in particular since it is cost- and time- effective.

### Explant preparation

Preferably, the primary shoot explants used in the present invention are obtained from healthy seedling's stock produced *in vitro* from seeds imbibed in ascorbic acid solution (100-200 mg/l) prior to germination. Axillary shoots developed *in vitro* from the basal meristematic tissue of the primary shoot explants can also be used for further shoot multiplication. This invention eliminates the need of further sterilisation of the explant and is cost- and time- effective.

In some preferred embodiments of obtaining primary shoot explants, the roots of some seedlings were completely removed to expose the basal meristem while those of other seedlings were cut 2-5 mm below the basal plate. In most preferred embodiments, the apical dome of a primary shoot explant is mechanically removed with a scalpel by making a nick, above the basal plate while retaining the leaf sheaths. Shoots with apical dome retained served as a control. The removal of both the roots of a seedling and the apical dome of a shoot explant were performed aseptically in a laminar flow cabinet, under a solution of, but not limited to, ascorbic acid (100-200 mg/l) singly or in combination with PVP (0.5%-1%) to prevent the browning of the exposed wounded plant tissues which has been reported to be one of the major constrains for achieving a successful *in vitro* culture of *Strelitzia.*

Although the effect of the mechanical removal of the apical dome on induction of axillary shoots was tested *in vivo* on mature plant, the procedure did not prove commercially viable due to the slow regeneration rate of the plantlets and seasonal and developmental variations in growth responses. However, there is no report on the successful application of such procedure *in vitro* due to the strong oxidative browning of *Strelitzia's* tissue in culture. The present invention for a first time describes a modified procedure of the mechanical removal of the apical dome of shoot explants using young seedlings as a plant source material. All procedures involving the mechanical removal of the root and the apical dome, and in the sub-culturing of the explants are performed under a solution of antioxidant which prevents/reduces substantially the oxidative browning of wounded plant tissues and improves the surviving rate of explants in *in vitro* culture.

### Culture of explants for shoot induction

The shoot explants with removed apical dome were cultured on shoot induction media comprising of MS basal media containing full strength of sucrose (30 g/l), 2-3 g/l of Gelrite and PGRs such as 6-benzylaminopurine (BAP) alone or in combinations with indole-3-butyric acid (IBA) and α-naphthaleneacetic acid (NAA).

The concentrations and combinations of plant growth regulators used in this invention for *in vitro* axillary shoot induction are presented in Table 2. The *in vitro* produced axillary shoots served as an explant for further subcultures on shoot induction media.

**Table 2. Concentrations and combinations of plant growth regulators for in vitro shoot induction**

| **PGR Treatment** | **Concentration** |
|---|---|
| BAP | 1 mg/l |
| | 2 mg/l |
| | 4 mg/l |
| BAP | 2 mg/l |
| + | + |
| Kin | 1 mg/l |
| BAP | 2 mg/l |
| + | + |
| IBA | 1 mg/l |
| BAP | 2 mg/l |
| + | + |
| NAA | 1 mg/l |
| BAP | 2 mg/l |
| + | + |
| IBA | 1 mg/l |
| + | + |
| NAA | 1 mg/l |

The effects of antioxidants but not limited to, PVP and activated charcoal, on the prevention of browning of shoot cultures, were also tested by culturing the explants on MS media supplemented with 0.5-2% of PVP or activated charcoal.

### Culture of shoot elongation

In some embodiments the axillary buds and some shoots (smaller than 2-3 cm in length), developed on shoot induction media, were first sub-cultured on a PGR- free MS basal media, containing vitamins, full strength sucrose (30g/l) and Gelrite (0.2-0.3%) for 2-3 weeks, for elongation, prior the transfer to a solid or liquid root induction media.

### Transfer to rooting

In some embodiments, the *in vitro* produced clusters of axillary shoots (longer than 3 cm in length) and/or the elongated shoots, were separated and cultured individually on a solid or liquid MS rooting media supplemented with root-inducing PGRs such, as but not limited to indole-3-butyric acid (IBA) in the range of 1-5 mg/l.

The effect of activated charcoal, PVP and frequent sub-culturing on prevention of browning of the cultures and on root induction were tested. In some embodiments, the rooting media may contain, but not limited to, 05%-2% activated charcoal, PVP or salicylic acid.

### Culture Conditions

Seed germination cultures were preferably maintained in a growth room at temperature 25°C, and 24 hours darkness. All seedling and shoot cultures were maintained in a growth room at temperature 25°C, photoperiod of 14-16 hours and light intensity of 50-90 µmol m⁻²s⁻¹. Growth responses of cultures such as shoot or root induction were observed on a weekly basis. In certain embodiments, the culture conditions (i.e. photoperiod, light intensity, growth media, temperature, relative humidity) are the same throughout the germination, initiation, and elongation, and rooting stages. Sub-culturing was performed as necessary.

### Acclimatisation

After the establishment of well-formed roots, *in vitro* regenerated plantlets were removed from cultures, roots were washed with sterile distilled water and transferred to jars filled with sterile vermiculite. Jars were covered with transparent plastic bags to provide sufficient light and humidity during the initial stage of the acclimatisation To acclimatise *in vitro* produced plants, holes were made on plastics bags on a weekly basis to reduce the humidity and gradually expose plants to the lower humidity of the surrounding environment. Acclimatised plants were transferred to pots filled with potting mixture of, but not limited to, potting soil, vermiculite and sand (2:1:1) and kept in a growth room at 25°C, photoperiod of 14-16 hours and light intensity of 150-200 µmol m⁻²s⁻¹up to 24 months. Plants were watered twice a week with water and alternated by a Hoagland nutrient solution as cited in Taiz and Zeiger (2002).

The present invention provides methods for efficient *in vitro* seed germination and mass *in vitro* culture of *Strelitzia* using shoot explants derived from, but not limited to aseptically produced seedlings.

### EXAMPLE 1: Seed culture

### Seed germination

Steps in most preferred embodiment involved: mechanical removal of the aril (Figure 1 A) from the seed, scarification of seeds in 98.99% sulphuric acid for 25 minutes, washing seeds several times with dH₂O for removal of the seed coat debris, sterilisation of seeds (as described), culturing of seeds on PGR-free germination media comprising of MS culture media supplemented with half strength sucrose concentration (15 g/l) and 2-3 g/l Gelrite. The germinated seed are depicted in Figure 1B. The results of this study showed that the scarification and the position of the seeds on the culture media affect seed germination and seedling development in *vitro.* In most instances seeds did not germinate if not scarified (Figure 2 A), or only germinated at low rate without seedling development, if scarified but positioned vertically, with the hilum embedded, into the media (Figure2 B). In the most preferred embodiment of this invention, scarified seeds were positioned with the hilum above the culture media resulting in up to 90% germination over a period of two to three weeks (Figure 2 C). This procedure is 3-4 folds faster than conventional propagation by seeds, which can take up to six to twelve weeks, and has a high success rate considering the high cost of *Strelitzia* seeds.

### Seedling development

In most preferred embodiment for seedling establishment, cultures with germinated seeds were transferred from darkness to light conditions with photoperiod of 14-16 hours and light intensity of 50-90 µmol m⁻²s⁻¹ for about 4-5 weeks (Figure 3 A). The seedlings derived from this procedure may serve as an explant source for multiple shoot induction. The *in vitro* produced seedlings may remain healthy and viable in the germination culture, without sub-culturing, up to 6 months (Figure 3 B) thus providing healthy plant stock for a long period of time at lower cost since no frequent sub-culturing is required. This procedure has a potential for commercial application since it is cost- and time-efficient, and ensures high germination percentage and high success rate of seedling production in a short period of time.

In another preferred embodiment imbibition of seeds for 6-24 hours in AA (100-200 mg/ml) prior to germination resulted in better developed seedlings with strong basal part which were more suitable as a source of obtaining shoot explant for the mechanical removal of the apical dome (Figure 4, C-E) as compared to the seedlings developed from non-imbibed seeds or seeds imbibed in distilled water (Figure 4, A and B, respectively). The seedlings produced by this method of the invention may be suitable as an explant source for multiple shoot induction.

In another embodiment, where scarified and sterilised seeds were germinated in, culture-less, sterile plastic vessels lined with sterile wet tissue under dark or light conditions (50-60 µmol m⁻²s⁻¹) (Figure 5) can be even more cost- and time- effective for seed germination and aseptic seedling production (no culture media is required) as compared to the seedlings produced on MS culture. It appears that germination of *Strelitzia* seeds in plastic vessels is not affected by light. However, if seeds are germinated in glass culture vessels, cultures should be kept in darkness till protrusion of the radicle since light seems to inhibit germination in glass vessels.

To the Applicant's best knowledge, there are no reports on successful germination and seedling production of *Strelitzia in vitro* on culture media or under aseptic conditions without culture media. Some studies have used isolated embryos for *in vitro* culture with low success rate due to oxidative browning. The methods used in this invention provide commercially viable procedures for rapid and high success rate of seed germination and seedling development which also may serve as an explant source for *in vitro* multiplication. This invention could be beneficial for large scale production of *Strelitzia* spp. and S. *juncea* in particular.

The Applicant's invention therefore presents an unexpected, novel and inventive solution when compared to the teaching and results in the prior art.

### EXAMPLE 2: Shoot culture in vitro

### Explant preparation

Preferably, the primary shoot explants used in the present invention (Figure 6) are obtained from healthy seedlings produced *in vitro* from seeds imbibed in ascorbic acid solution (100-200 mg/l) prior to germination. (Figures 4 and 5). Axillary shoots developed *in vitro* from the basal meristematic tissue of the primary shoot explant may also be used as an aseptic explant source for further induction of multiple axillary shoots thus resulting in higher multiplication rate over a shorter period of time as compared to *in vivo* multiplication (Figures 8, 9 and 10). This invention eliminates the need of sterilisation of the explant source and is more cost and time effective.

In the most preferred embodiments, the apical dome of a seedling is mechanically removed with a scalpel by making a nick above the basal plate while retaining the leaf sheaths of the seedling (Figure 6B and 6C). The removal of the apical dome and the roots were performed aseptically in a laminar flow cabinet, under a solution of, but not limited to, ascorbic acid (100-200 mg/l) singly or in combination with PVP (0.5%-1%) to prevent the oxidative browning of the exposed wounded surfaces which has been reported to be one of the major constraints for achieving a successful *in vitro* culture of *Strelitzia.*

Although the effect of the mechanical removal of the apical dome on induction of axillary shoots was tested *in vivo,* on mature plants, the procedure did not prove to be commercially viable due to the slow regeneration rate of the plantlets and seasonal, and developmental variations in growth responses. However, there is no report on the successful application of such procedure *in vitro* due to the strong oxidative browning of *Strelitzia's* tissues in culture. The present invention, for a first time, describes a modified procedure of the mechanical removal of the apical dome (making a nick above the basal plate) using young seedlings as a source of explant to isolate the basal meristem while cutting the plant tissues under a solution of antioxidant to prevent the exposure of the wounded surfaces to the oxygen. This procedure reduces substantially the oxidative browning of the explants in culture which improves the surviving rate of *Strelitzia* explants in *in vitro* culture.

### EXAMPLE 3: Axillary shoot induction

The explants with removed apical dome were cultured on MS media containing full strength of sucrose (30 g/l), 2-3 g/l of Gelrite and various concentrations of PGR such as 6-benzylaminopurine (BAP) alone or in combinations of indole-3-butyric acid (IBA) and/or α-naphthaleneacetic acid (NAA).

In one embodiment, a culture of primary shoot explants on MS media supplemented with 2 mg/l BAP and 1 mg/l IBA raised few primary axillary shoots per explant. Splitting and subculturing of these shoots (Figure 7) on a fresh MS media with the same PGR combination resulted in a rapid (2-3 weeks) induction of numerous, about but not limited to 12-15, secondary axillary buds/shots which may further establish into well-developed shoots over a period of 8-10 weeks with or without subculturing to fresh media (Figure 8).

The cluster of axillary shoots was separated in a solution of ascorbic acid (100-200 mg/l), using the procedure formulated in this invention. The small axillary shoots (less than 3 cm in length) (Figure 9 A) or cluster of buds together with an established shoot (Figure 10), were sub-cultured on a hormone free culture media for elongation while larger explants (Figure 9 B) were transferred to rooting media.

All *in vitro* obtained axillary shoots can also serve as a secondary or tertiary shoot explants for further axillary shoot multiplication *in vitro* following the procedure described by this invention.

In another embodiment, it was possible to induce the proliferation of numerous third generation of axillary shoots (Figure 10) on secondary axillary shoot explant obtained from previous cultures (Figure 9 B) when-sub cultured on the culture medium formulated in the invention (MS basal culture medium, 2.0 mg/l BAP and 1.0 mg/l IBA, 2-3 g/l Gelrite and 30 g/l sucrose) for 6-8 weeks. This procedure has high multiplication potential for a clonal propagation since it may result in total production of about, but not limited to 45 axillary shoots per explant from six or more subcultures and established shoots may remain up to 12-14 weeks without sub-culturing which is cost-effective.

In yet another embodiment, the elongation of axillary buds/shoots was carried out by subculture on PGR-free media comprising of MS basal culture medium, 2-3 g/l Gelrite and 30 g/l sucrose in a presence or absence of activated charcoal (Figure 11) The presence of an established shoot as part of the cluster of buds improved the survival of the buds and their subsequent elongation on PGR free media (Figure 10).

### EXAMPLE 4: Induction of root development

Adventitious roots were induced at the base of, *in vitro* obtained, axillary shoots on media comprising of MS basal culture medium, 1-2 mg/l IBA, 2-3 g/l Gelrite, and 30 g/l sucrose (Figure 12 A). There is no root formation if the cut on the shoot explant is few mm below the basal meristem (Figure 12 B). Figure 13 (A and B) displays the *Strelitzia* plantlets regenerated *in vitro* using the procedures developed in this invention.

Examples of different stages of development of acclimatised *Strelitzia juncea* plants regenerated *in vitro* according to this invention, after the transplanting to soil comprising of a 2:1:1 mixture of potting soil, vermiculate and sand are displayed in Figure 14: Plants at juvenile stage of development with broad leaves (A and B) kept in soil mixture for 3-4 months and 12-13 months respectively. Figure 14C and D display adult stage of plants, kept for (C) 24 and (D) 30 months in soil, with smaller leaves on top and formation of a rush with subsequent subdivision between the middle leaves of the fan.

The Applicant's invention therefore provides a method for *in vitro* propagation of *Strelitzia,* said method resulting in a high multiplication rate. The method is therefore suitable for large scale production.

### EXAMPLE 5: Induction of shoot and root development in one culture

In another embodiment both shoots and roots were induced after culturing shoot explants with removed apical dome on MS basal medium with 2 mg/l BAP and 1 mg/l NAA, 2-3 g/l Gelrite and 30 g/l sucrose (Figure 15B).

In another embodiment, *in vitro* development of both axillary shoots and adventitious roots were induced on axillary shoot explants, derived from previous cultures (Figure 16A and B) and after subculture on media comprising of MS basal culture medium, PGRs (2 mg/l BAP, and 1 mg/l NAA), 2-3 g/l Gelrite, and 30 g/l sucrose (Figure 16C).

In yet another embodiment (Figure 17), it was possible to proliferate new secondary axillary shoots and roots on axillary shoot explant obtained from previous cultures after subculture on the same media for 3-4 weeks. In this embodiment, the proliferation of axillary shoots occurred even in the presence of some blackening on the wounded plant surfaces since the tissues beneath the blackening remained healthy and responsive to exogenously applied PGR in further subcultures (Figure 17 A and B). The black sheet-like cover can be easily removed under the antioxidant solution to prevent browning of the wounded surfaces prior to culture. The media formulated in the invention (comprising of MS basal culture medium, PGR (2 mg/l BAP, and 1 mg/l NAA), 2-3 g/l Gelrite, and 30 g/l sucrose) induced, in addition to shoot proliferation, also root formation which seemed to limited the contact of the wounded stem tissues with the culture media and thus preventing/reducing blackening of *Strelitzia* wounded tissues in cultures which is detrimental for the success of its micro-propagation (Figure 17 C). Figure 17 displays examples of acclimatised *Strelitzia* plants regenerated *in vitro* according to this invention after the transfer to a soil mixture comprising of a 2:1:1 of potting soil, vermiculite and sand for: (D) 2-3 months and (E) 12-13 months.

### EXAMPLE 6: Effect of combination of three PGRs on in vitro axillary shoot development and growth

In another embodiment, multiple axillary buds and shoots were induced after culturing of shoot explants with removed apical dome on MS culture containing three combinations of PGR such as 2 mg/l BAP and 1mg IBA, and 1 mg/l NAA. Lower number of axillary shoots per explant was raised in this media but faster growth and root regeneration were observed (Figure 18A and Figure 18 B). Elongation and new axillary shoot proliferation occurred after one-two weeks of subculture on shoot induction media (Figure 18C and D). Induction of root development on the base of axillary shoots may occur in some instances on the same culture media in the presence of auxins (IBA and NAA) or can be carried out by sub-culturing the axillary shoots on a rooting MS culture medium supplemented with 1 mg/l IBA (E).

Figure 19 displays examples of acclimatised *Strelitzia* plants regenerated *in vitro* according to this procedure of the invention, after their transfer to soil comprising a 2:1:1 mixture of potting soil, vermiculated and sand.

### EXAMPLE 7: Effect of using a single PGR for multiple buds and shoot induction

In another embodiment, multiple axillary buds and shoots were induced after the explants with removed apical dome were cultured on MS culture containing different concentrations of a single PGR such as 1 mg/l BAP (Figure 20 and Figure 21) and 2 mg/l BAP (Figure 22). In this embodiment, a rapid proliferation of multiple, but weaker, axillary shoots and continues multiplication from *in vitro* produced axillary shoots was achieved after few weeks by using the procedure described in this invention.

### EXAMPLE 8: Effect of PGRs on multiple shoot induction from in vitro germinated seeds

In another embodiment multiple shoots were induced from seeds imbibed in ascorbic acid for 12-24 hours and germinated *in vitro* on culture media comprising of MS basal media, vitamins, 15 g/l sucrose and PGRs such as cytokinin (BAP) alone or in combination with auxins (IBA and NAA). Multiple shoot induction directly from seeds is depicted on Figure 23 (B-D). The presence of auxins (NAA or/and IBA) in combination with BAP resulted in development of strong seedlings with broader base and multiple shoots and roots (Figure 23C, D and E) as compared to seedlings developed in the presence of BAP alone or in the absence of PGRs (Figure 23B and A, respectively). The highest number of multiple shoots were induced in the presence of three PGR combinations (2 mg/l BAP + 1 mg/l IBA +1 mg/l NAA), Figure 23D. Multiple shoots of these seedlings may serve as an explant source for further multiplication. This procedure has a potential for commercial application since it is time- and cost- effective for *Strelitzia's* multiplication *in vitro.*

The Applicant's invention provides for use of the methods described in large scale mass production of *Strelitzia* species.

### REFERENCES

Aasim, M., Khawar, K.M., Ozcan, S., 2009. Comparison of shoot regeneration on different concentrations of thidiazuron from shoot tip explant of cowpea on Gelrite and agar containing medium. Notulae Botanicae Horti AgrobotaniciCluj-Napoca Cluj-Napoca 37, 89-93.
Badoni, A., Chauhan, J.S., 2010. In Vitro Sterilization Protocol for Micropropagation of Solanum tuberosum cv. 'Kufri Himalini'. Academia Arena 2, 24-27.
Baes, P.O., Viana, M.L., Suhring, S., 2001. Germination in Prosopis ferox seeds: effects of mechanical, chemical and biological scarificators. Journal of Arid Environments 50, 185-189.
Barbosa, J.G., Mantovanialvarenga, E., Fernandes Dos Santos Dias, D.C., Vleira, A.N., 2005. Effect of acid scarification and different temperatures on Physiological quantity of Strelitzia reginae seeds. Revista Brasileria de sementes 27, 71-77.
Bonga, J.M., Klimaszewska, K.K., Von Aderkas, P., 2010. Recalcitrance in clonal propagation, in particular of conifers. Plant Cell Tissue Organ Culture 100, 241-254.
Burgess, C., 2004. Bird of Paradise. Home and garden Information Centre. HGIC 1562. Clemson Extension, Clemson University. Avalaible at http://hgic.clemson.edu/ [Accessed 08 February 2012].
Casal, J.J., Sanchez, R.A., Botto, J.F., 1998. Modes of action of phytochromes. Journal of Experimental Botany 49, 127-138.
De Meillon, S., Van De Venter, H.A., Small, J.G.C., 2002. The respiratory metabolism of Strelitzia juncea Ait. seeds. The effect of dormancy release by oxygen on certain glycolytic enzyme activities and metabolite concentrations. Available at http://jxb.oxfordjournals.org/cgi/content/abstract/41/7/885 [accessed 10 March 2010].
Dixon, R.A, Gonzales, R.A., 1995. Plant Cell Culture: A Practical Approach 2nd Ed. Oxford University Press, New York, USA, pp 12-15.
DuPont, S.T., 2011. Seed and Seedling Biology. Penn State Extension: College of Agricultural Science 5, 1-6.
Emek, Y., Erdag, B., 2007. In vitro propagation of Gladiolus anatolicus (Boiss.) stapf. Pakistan Journal of Botany 39, 23-30.
Fernandez, A.M.A, Escutia, J.L.P., Mancera, H.A.Z., 2008. Somatic proembryo induction in bird of paradise (Strelitzia reginae Banks). Rev. Fitotee.mex 31, 183-186.
Finch-Savage, W.E., Leuber-Metzger, G., 2006. Seed dormancy and control of germination. New Physiologist 171, 501-523.
Hartmann, H.T., Kester, D.E., Davies JR, F.T., Geneve, R.L., 2002. Plant Propagation: Principles and Practices. 7th ed. Pearson Education, Inc. New Jersey, USA, pp 585-586.
Hensley, D., Deputy, J., Yogi, J., Leonhardt, K., 1998. Bird-of- paradise. Ornamentals and Flowers. College of Tropical Agriculture and Human Resources, 27.
Holley, D., 2009. Plant cuttings in Agriculture and Horticulture: investigating the Applications of vegetative propagation. Available at http://www.suite101.com/content/plant-cuttings-in-agriculture- and-horticulturea142498 [accessed 05 august 2011].
Jatt, T., Suhail, M., Abro, H., Larik, A.S., 2007. Alleviating seed dormancy of Tectona grandis L. by temperature, Plant growth regulators and inorganic salts. Pakistan Journal of Botany 39, 2581-2583.
Kantharaju, M., Kulkarni, B.S., Reddy, B.S., Hedge, R.V., Patil, B.C., Adiga, J,D., 2008. Effect of antioxidant on in vitro establishment of Strelitzia reginae through shoot tip explants. Karnataka Journal of Agricultural Science, 21 (2), 324-325.
Madhusudhanan, K., Rahiman, B.A., 2000. The effect activated charcoal supplemented media to browning of in vitro cultures of Piper species. Biologia Plantarum, 43, 297-299.
Murashige, T., Skoog, F., 1962. A revised medium for rapid growth and bio assays with tobacco tissue cultures. Physiologia Plantarum 5, 473-497.
North, J.J., Ndakimedi, P.A., Laubscher, C.P., 2010. The potential of developing an in vitro method for propagating Strelitziaceae. African Journal of Biotechnology 9, 7583-7588.
North, J.J., Ndakimedi, P.A., Laubscher, C.P., 2011. Effects of various media compositions on the in vitro germination and discoloration of immature embryos of bird of paradise (Strelitzia reginae). Plant Omics Journal 4, 100113.
Notten, A., 2002. Strelitzia reginae "Mandela's gold". South African National Biodiversity Institute (SANBI). Available at http://www.plantzafrica.com/plantgrs/strelitegmandelagold.htm [accessed 08 February 2012].
Ozel, C.A., Khawar, K.M., Arslan, O., 2008. A comparison of the gelling of isubgol, agar and Gelrite on in vitro shoot regeneration and rooting of variety Samsun of tobacco (Nicotiana tabacum L.). Scientia Horticulturae 117, 174-181.
Paiva, P.D.O., Paiva, R., Pasqual, M., Paiva, L.V., 2004. In vitro establishment of Strelitzia (Strelitzia reginae Banks.). Ciênce Agrotecnol 28, 1031-1037.
Pan, M.J., Van Staden, J., 1998. The use of charcoal in vitro culture-A review. Plant Growth Regulation 26,15-163.
Pati, P.K., Rath, S.P., Sharma, M., Sood, A., Ahuja, P.S., 2005. In vitro propagation of rose: a review. Biotechnology Advances, 94-111.
Pirone, C., Johnson, J.V., Quirke, M.E., Priestap, H.A., Lee, D., 2010. The animal pigment bilirubin identified in Strelitzia reginae, the bird of paradise flower Hortscience 45(9):1411-1415.
Pogroszewska, E., 2006. The effect of scarification, gibberellic acid and ethephon on seeds germination and the growth of Strelitzia reginae seedlings. Institute of Ornamental Plants and Landscape Architecture, Agricultural University, Lublin, Poland 510, 479-489. Available at http://psjc.icm.edu.pl/psjc/cgi-bin/getdoc.cgi?AAAA017072 [accessed 15 February 2010].
Preece, J.E., Read, P.E., 2005. The biology of horticulture: an introductory text book 2nd Ed. John Wiley and sons, Inc. New Jersey, U.S.A, pp. 373-375.
Sayers, S., 2007. Strelitzia parvifolia var. juncea. Sayers Strelitzia Nursery. Available at http://www.sayers-strelitzia.com.au/juncea.html [accessed 24 August 2011].
Shanmugavalli, M., Renganayaki, P.R., Menaka, C., 2007. Seed dormancy and germination improvement treatments in fodder sorghum. eJournal ICRISAT 3, 1-3.
Singh, A.K., 2006. Flower Crops: Cultivation and Management. New India publishing. New Delhi, pp. 45.
Shreevatsa, K.S., Jayaprasad, K.V., Narayanaswamy, P., Satyanarayana B.N., 2004 Establishing in vitro cultures of Strelitzia reginae using shoot tip explants. Journal of Ornamental Horticulture 7(3&4):431-434.
Steward, C.N., 2008. Plant Biotechnology and genetics: principle, techniques and applications. John Wiley and Sons, Inc. Hoboken, New Jersey pp 91, 115-120
Taiz, L., Zeiger, E., 2002. Plant Physiology. Sinauer Associates, Inc, Sunderland, U.S.A, pp. 423-429.
Van de Pol, P.A., Van Hell, T.F., 1988. Vegetative propagation of Strelitzia reginae. ISHS Acta Horticulturae 226: International Symposium on propagation of ornamental plants.
Van der Walt, L., 2000. Strelitzia reginae banks. South African National Biodiversity Institute(SANBI). Available at http://www.plantzafrica.com/plantgrs/strelitziareginae.htm [accessed 22 February 2010].
Vasudevan, R., Van Staden, J., 2010. In vitro asymbiotic seed germination and seedling growth of Ansellia africana Lindl. Scientia Horticulturae 123, 496-504.
Winter, J., 2004. Strelitzia juncea Link. South African National Biodiversity Institute (SANBI). Available at http://www.plantzafrica.com/plantgrs/strelitzjun.htm [accessed 22 February 2010].
Yang, Q.H., Ye, W.H., Yin, X.J., 2007. Dormancy and germination of Areca triandra seeds. Scientia Horticulturae 113, 107-111.

## Claims

1. An *in vitro* method for the propagation of a *Strelitzia* species from a *Strelitzia* seed, the method including the steps of sequentially:
(a) chemically scarifying the *Strelitzia* seed;
(b) thereafter, effecting surface sterilisation of the scarified seed;
(c) imbibing the scarified and sterilised seed in an anti-oxidant solution;
(d) inoculating the scarified and sterilised seed on germination media and allowing the seed to germinate in darkness to establish a germinated seed culture;
(e) in a seedling development step, subjecting the germinated seed culture to light conditions for a suitable photoperiod for seedling development;
(f) obtaining a shoot explant by removing the root of the seedling;
(g) mechanically removing an apical dome of the shoot explant while retaining a leaf sheath of the shoot explant, thereby uplifting apical dominance in the shoot explant;
(h) culturing the shoot explant with the removed apical dome on basal culture media containing a plant growth regulator, thereby initiating multiple shoot development on the shoot explant with the removed apical dome, or
when the germination media comprises basal culture media supplemented with a plant growth regulator, culturing the scarified and sterilised seed directly, thereby initiating multiple shoot development;
(i) separating the resulting multiple shoots and, if required, subculturing axillary shoots to shoot induction media for further multiplication;
(j) transferring axillary shoots to root development media for inducing root growth, and hence plantlet regeneration;
(k) acclimatising the resultant *in vitro* produced plantlets.

2. The method of Claim 1, wherein the *Strelitzia* species is *Strelitzia juncea* or *Strelitzia reginae.*

3. The method of Claim 1 or Claim 2, wherein:
(a) said method includes the step of mechanically removing the aril from the seed, before scarification thereof, and/or
(b) the chemical scarification of the seed is carried out by incubating the seed in concentrated sulphuric acid.

4. The method of any one of Claims 1 to 3, wherein the seed germination media comprises:
(a) full strength, plant growth regulator (PGR)-free, Murashige and Skoog's (MS) basal media with vitamins, reduced level of sucrose (7.5-15 g/l) and Gelrite (2-3 g/l) as gelling agent; or
(b) full strength Murashige and Skoog's (MS) basal media with vitamins, reduced level of sucrose (7.5-15 g/l), Gelrite (2-3 g/l) as gelling agent, and 6-benzylaminopurine (BAP) alone (1-2 mg/l) or in combination with 1 mg/l indole-3-butyric acid (IBA) and/or 1mg/l α-naphthaleneacetic acid (NAA) as plant growth regulator (PGR).

5. The method of any one of Claims 1 to 4, wherein the inoculation of the seed on the seed germination media is carried out by placing the seed on the media, with a hilum of the individual seeds positioned above the media, wherein the seed is allowed to geminate for 2-3 weeks in darkness at 25°C.

6. The method of any one of Claims 1 to 5, which includes imbibition of the seed in an anti-oxidant solution before the seed germination step, thereby resulting in better seedling development relative to seedling derived from seed imbibed in distilled water or non-imbibed seed, wherein the anti-oxidant solution is an ascorbic acid solution containing 100-200 mg/l of ascorbic acid, and wherein the time of imbibition is about 12-24 hours.

7. The method of any one of Claims 1 to 6, wherein the seedling development includes incubating the seedling cultures, under controlled conditions at about 25°C, for a photoperiod of 12-16 hours and a light intensity of about 50-90 µmol m⁻²s⁻¹, and wherein the seedling development is effected for about 4 to about 7 weeks.

8. The method of any one of Claims 1 to 7, wherein
(a) the obtaining of aseptic shoot explants from seedling is effected by the mechanical removal of the root below the basal plate of the seedling; and/or
(b) when the uplifting of the apical dominance of a shoot explant is effected by mechanical removal of the apical dome, a leaf sheath of the shoot explant is retained, and wherein the mechanical removal of the apical dome is carried out by making a nick above a basal plate of the seedling.

9. The method of Claim 8, wherein the removal of the root of a seedling and the removal of the apical dome of a shoot explant are performed while the seedling and/or the shoot are immersed in an anti-oxidant solution, wherein the anti-oxidant solution comprises:
(a) an ascorbic acid solution (100-200 mg/l); or
(b) an ascorbic acid solution mixed with polyvinyl-pyrrolidone (PVP), thereby to prevent browning of the cultured explants.

10. The method of any one of Claims 1 to 9, wherein the initiation of the multiple shoot development on the shoot explant with removed apical dome is carried out and is effected by culturing the shoot explant with the removed apical dome on MS basal media containing vitamins, full strength sucrose (30 g/l), Gelrite (2-3 g/l) and at least one shoot inducing plant growth regulator wherein the plant growth regulator is 6-benzylaminopurine (BAP) alone (1-4 mg/l) or in combination with 1 mg/l indole-3-butyric acid (IBA), and/or in combination with 1 mg/l α-naphthaleneacetic acid (NAA), or in combination with 1 mg/l kinetin.

11. The method of any one of Claims 1 to 10, wherein initiation of adventitious roots is effected by culturing the shoot explant on root induction medium, wherein the root induction media is solid or liquid MS culture medium.

12. The method of Claim 11, wherein either;
(a) solid root-induction media is used, and comprises MS basal culture medium, PGR auxin (1-5 mg/l IBA or/and NAA), Gelrite (2-3 g/l) and 30 g/l sucrose; or
(b) liquid root-induction medium is used, and comprises only of MS basal culture medium and PGR auxin (1-5 mg/l IBA or/and NAA).

13. The method of any one of Claims 1 to 12, wherein shoot growth and root development are both simultaneously induced on the shoot explant with the removed apical dome, and wherein the simultaneous shoot growth and root development induction are effected by culturing the shoot explant in culture media containing combinations of 1-2 mg/l shoot inducing PGR (BAP) and 1-2 mg/l root inducing PGR (NAA).

14. The method of any one of Claims 1 to 13, wherein the initiation of the multiple shoot development or induction by culturing the scarified and sterilised seed directly is carried out, and is effected on seed germination media comprising MS basal medium, vitamins, half strength sucrose (15 g/l), 2-3 g/l Gelrite and 6-benzyl aminopurine (BAP) alone (1-2 mg/l) or in combination with 1 mg/l indole-3-butyric acid (IBA) or/and 1mg/l α-naphthaleneacetic acid (NAA).

15. An *in vitro* method for rendering a *Strelitzia* seed amenable to germination, the method including the steps of sequentially:
(a) chemically scarifying the *Strelitzia* seed;
(b) thereafter, effecting surface sterilisation of the scarified seed;
(c) imbibing the scarified and sterilised seed in an anti-oxidant solution; and
(d) placing the scarified and sterilised seed on seed germination media, with a hilum of the seed positioned above the media and allowing the seed to germinate.

## Patentansprüche

1. In-vitro-Verfahren zum Vermehren einer Strelitzia-Spezies aus einem Strelitzia-Samen, wobei das Verfahren nacheinander die Schritte umfasst:
(a) chemisches Aufreißen bzw. Anritzen des Strelitzia-Samens,
(b) danach Vornehmen einer Oberflächensterilisierung des aufgerissenen bzw. angeritzten Strelitzia-Samens,
(c) Aufnehmen des aufgerissenen bzw. angeritzten und sterilisierten Samens in einer Antioxidanslösung,
(d) Okulieren des aufgerissenen bzw. angeritzten und sterilisierten Samens auf Keimungsmedien und Ermöglichen dem Samen, in Dunkelheit zu keimen, um einen gekeimten Samen herzustellen,
(e) in einem Keimlingsentwicklungsschritt Aussetzen des gekeimten Samens Lichtbedingungen während einer geeigneten Photoperiode zur Keimlingsentwicklung,
(f) Erzielen eines Schößling-Explantats durch Entfernen der Wurzel des Keimlings,
(g) mechanisches Entfernen einer apikalen Kuppe des Schößling-Explantats, während eine Blattscheide des Schößling-Explantats erhalten bleibt, wodurch die apikale Dominanz in dem Schößling-Explantat herausgehoben wird,
(h) Kultivieren des Schößling-Explantats mit der entfernten apikalen Kuppe auf Basalkulturmedien, die einen Pflanzenwachstumsregulator enthalten, wodurch eine Mehrfach-Schößlingsentwicklung an dem Schößling-Explantat mit der entfernten apikalen Kuppe initiiert wird, oder dann, wenn die Keimungsmedien Basalkulturmedien umfassen, welche mit einem Pflanzenwachstumsregulator ergänzt sind, direktes Kultivieren des aufgerissenen bzw. angeritzten und sterilisierten Samens, wodurch eine Mehrfach-Schößlingentwicklung initiiert wird,
(i) Trennen der resultierenden mehreren Schößlinge und, falls erforderlich, Subkultivieren von axillaren Schößlingen in schößling-induzierenden Medien zur weiteren Vermehrung,
(j) Übertragen der axillaren Schößlinge in Wurzelentwicklungsmedien, um ein Wurzelwachstum und folglich eine Pflänzchen-Regeneration zu induzieren,
(k) Akklimatisieren des resultierenden in vitro erzeugten Pflänzchen.

2. Verfahren nach Anspruch 1, wobei die Strelitzia-Spezies die Strelitzia juncea oder Strelitzia reginae ist.

3. Verfahren nach Anspruch 1 oder 2, wobei
(a) das Verfahren den Schritt des mechanischen Entfernens des Samenmantels von dem Samen vor dessen Aufreißen bzw. Anritzen enthält und/oder
(b) das chemische Aufreißen bzw. Anritzen des Samens durch Inkubieren des Samens in konzentrierter Schwefelsäure durchgeführt wird.

4. Verfahren nach einem der Ansprüche 1 bis 3, wobei die Samen-Keimungsmedien umfassen:
(a) Pflanzenwachstumsregulator-(PGR)-freie Murashige- und Skoog's-(MS)-Basalmedien voller Stärke mit Vitaminen, reduziertem Saccharosegehalt (7,5-15g/l) und Gelrite (2-3g/l) als Geliermittel oder
(b) Murashige- und Skoog's-(MS)-Basalmedien voller Stärke mit Vitaminen, reduziertem Saccharosegehalt (7,5-15g/l) Gelrite (2-3g/l) als Geliermittel und 6-Benzylaminopurin (BAP) allein (1-2mg/l) oder in Kombination mit 1mg/l Indol-3-Buttersäure (IBA) und/oder 1mg/l α-Naphtalen-Essigsäure (NAA) als Pflanzenwachstumsregulator (PGR).

5. Verfahren nach einem der Ansprüche 1 bis 4, wobei das Okulieren des Samens auf den Samen-Keimungsmedien ausgeführt wird, indem der Samen auf den Medien platziert wird, wobei ein Hilum der einzelnen Samen oberhalb der Medien positioniert wird, wobei dem Samen ermöglicht wird, während zwei bis drei Wochen in Dunkelheit bei 25°C zu keimen.

6. Verfahren nach einem der Ansprüche 1 bis 5, welches ein Aufnehmen des Samens in einer Antioxidanslösung vor dem Samenkeimungsschritt enthält, was zu einer besseren Keimlingsentwicklung bezogen auf einen Keimling führt, der von einem Samen, welcher in destilliertem Wasser aufgenommen ist, oder von nicht-aufgenommenem Samen stammt, wobei die Antioxidanslösung eine Ascorbinsäurelösung ist, die 100-200mg/l Ascorbinsäure enthält,
und wobei die Zeit der Aufnahme etwa 12-24 Stunden beträgt.

7. Verfahren nach einem der Ansprüche 1 bis 6, wobei die Keimlingsentwicklung ein Inkubieren der Keimlingskulturen unter kontrollierten Bedingungen bei etwa 25°C während einer Photoperiode von 12-16 Stunden und einer Lichtintensität von etwa 50-90µmol m⁻²s⁻¹ enthält
und wobei die Keimlingsentwicklung während etwa 4 bis etwa 7 Wochen hervorgerufen wird.

8. Verfahren nach einem der Ansprüche 1 bis 7, wobei
(a) das Gewinnen von aseptischen Schößling-Explantaten aus dem Keimling durch das mechanische Entfernen der Wurzel unter der Basalplatte des Keimlings bewirkt wird und/oder
(b) dann, wenn das Herausheben der apikalen Dominanz eines Schößling-Explantats durch mechanisches Entfernen der apikalen Kuppe vorgenommen wird, eine Blattscheide des Schößling-Explantats beibehalten wird, und wobei das mechanische Entfernen der apikalen Kuppe durch Vornahme einer Kerbe oberhalb einer Basalplatte des Keimlings durchgeführt wird.

9. Verfahren nach Anspruch 8, wobei das Entfernen der Wurzel eines Keimlings und das Entfernen der apikalen Kuppe eines Schößling-Explantats durchgeführt werden, während der Keimling und/oder der Schößling in eine Antioxidanslösung eingetaucht sind, wobei die Antioxidanslösung umfasst:
(a) eine Ascorbinsäurelösung (100-200mg/l) oder
(b) eine Ascorbinsäurelösung, die mit Polyinyl-Pyrrolidon (PVP) gemischt ist, um dadurch eine Bräunung der kultivierten Explantate zu verhindern.

10. Verfahren nach einem der Ansprüche 1 bis 9, wobei das Initiieren der Mehrfach-Schößlingsentwicklung an dem Schößling-Explantat mit entfernter apikaler Kuppe durch Kultivieren des Schößling-Explantats mit der entfernten apikalen Kuppe auf MS-Basalmedien durchgeführt und bewirkt wird, die Vitamine, Saccharose (30g/l) voller Stärke, Gelrite (2-3g/l) und zumindest einen schößling-induzierenden Pflanzenwachstumsregulator enthalten,
wobei der Pflanzenwachstumsregulator 6-Benzylaminopurin (BAP) allein (1-4mg/l) oder in Kombination mit 1mg/l Indol-3-Buttersäure (IBA) und/oder in Kombination mit 1mg/l α-Naphthalen-Essigsäure (NAA) oder in Kombination mit 1mg/l Kinetin ist.

11. Verfahren nach einem der Ansprüche 1 bis 10, wobei das Initiieren von hinzukommenden Wurzeln durch Kultivieren des Schößling-Explantats auf einem Wurzel-Induktionsmedium bewirkt wird, wobei die Wurzel-Induktionsmedien ein festes oder flüssiges MS-Kulturmedium sind.

12. Verfahren nach Anspruch 11, wobei entweder
(a) feste Wurzel-Induktionsmedien verwendet werden und ein MS-Basalkulturmedium, PGR-Auxin (1-5mg/l IBA oder/und NAA), Gelrite (2-3g/l) und 30g/l Saccharose umfassen oder
(b) ein flüssiges Wurzel-Induktionsmedium verwendet wird und lediglich ein MS-Basalkulturmedium und PGR-Auxin (1-5mg/l IBA oder/und NAA) umfasst.

13. Verfahren nach einem der Ansprüche 1 bis 12, wobei das Schößlingswachstum und die Wurzelentwicklung beide gleichzeitig an dem Schösslings-Explantat mit der entfernten apikalen Kuppe induziert werden
und wobei das gleichzeitige Schößlingswachstum und Induzieren der Wurzelentwicklung durch Kultivieren des Schösslings-Explantats in Kulturmedien bewirkt wird, die Kombinationen von 1-2mg/l schößling-induzierendem PGR (BAP) und 1-2mg/l wurzelinduzierendem PGR (NAA) enthalten.

14. Verfahren nach einem der Ansprüche 1 bis 13, wobei das Initiieren der Mehrfach-Schößlingsentwicklung oder -induktion durch direktes Kultivieren des aufgerissenen bzw. angeritzten und sterilisierten Samens ausgeführt wird und auf Samen-Keimungsmedien bewirkt wird, umfassend ein MS-Basalmedium, Vitamine, Saccharose halber Stärke (15g/l), 2-3g/l Gelrite und 6-Benzyl-Aminopurin (BAP) allein (1-2mg/l) oder in Kombination mit 1mg/l Indol-3-Buttersäure (IBA) oder/und 1mg/l α-Naphtalen-Essigsäure (NAA).

15. In-Vitro-Verfahren, um einen Strelitzia-Samen einer Keimung zugänglich zu machen, wobei das Verfahren nacheinander die Schritte enthält:
(a) chemisches Aufreißen bzw. Anritzen des Strelitzia-Samens,
(b) danach Bewirken einer Oberflächensterilisierung des aufgerissenen bzw. angeritzten Samens,
(c) Aufnehmen des aufgerissenen bzw. angeritzten und sterilisierten Samens in einer Antioxidanslösung und
(d) Aufbringen des aufgerissenen bzw. angeritzten und sterilisierten Samens auf Samen-Keimungsmedien, wobei ein Hilum des Samens oberhalb der Medien positioniert wird und dem Samen ermöglicht wird, zu keinem.

## Revendications

1. Procédé in vitro pour la propagation d'une espèce *Strelitzia* à partir d'une graine de *Strelitzia,* ledit procédé comprenant les étapes séquentielles consistant à :
(a) scarifier chimiquement les graines de *Strelitzia* ;
(b) ensuite, effectuer une stérilisation en surface des graines scarifiées ;
(c) imbiber les graines scarifiées et stérilisées d'une solution antioxydante ;
(d) inoculer les graines scarifiées et stérilisées sur un milieu de germination et laisser germer les graines dans l'obscurité pour constituer une culture de graines germées ;
(e) dans une étape de développement des semis, soumettre la culture de graines germées à des conditions de lumière pendant une photopériode appropriée pour le développement des semis ;
(f) obtenir un explant de pousse en enlevant la racine des semis ;
(g) retirer mécaniquement un dôme apical de l'expiant de pousse tout en gardant une gaine foliaire de l'expiant de pousse, soulevant ainsi la dominance apicale dans l'expiant de pousse ;
(h) cultiver l'expiant de pousse ayant le dôme apical retiré sur un milieu de culture de base contenant un régulateur de croissance végétale, initiant ainsi le développement de pousses multiples sur l'expiant de pousse ayant le dôme apical retiré, ou
lorsque le milieu de germination comprend un milieu de culture de base supplémenté avec un régulateur de croissance végétale, cultiver directement la graine scarifiée et stérilisée, initiant ainsi le développement de pousses multiples ;
(i) séparer les pousses multiples résultantes et, si nécessaire, repiquer les pousses axillaires sur un milieu d'induction de pousses pour la multiplication ultérieure ;
(j) transférer les pousses axillaires sur un milieu de développement de racines pour induire la croissance des racines, et donc la régénération des plantules ;
(k) acclimater les plantules résultantes, produites in vitro.

2. Procédé selon la revendication 1, dans lequel l'espèce *Strelitzia* est *Strelitzia juncea* ou *Strelitzia reginae.*

3. Procédé selon la revendication 1 ou la revendication 2, où (a) ledit procédé inclut l'étape consistant à retirer mécaniquement l'arille des graines, avant leur scarification, et/ou (b) la scarification chimique des graines est réalisée en faisant incuber les graines dans de l'acide sulfurique concentré.

4. Procédé selon l'une quelconque des revendications 1 à 3, dans lequel le milieu de germination des graines comprend :
(a) un milieu de base de Murashige et Skoog (MS) non dilué, dépourvu de régulateur de croissance végétale (PGR, pour « Plant Growth Regulator »), avec des vitamines, un niveau réduit de saccharose (7,5-15 g/l) et du Gelrite (2-3 g/l) comme agent gélifiant; ou
(b) un milieu de base de Murashige et Skoog (MS) non dilué, avec des vitamines, un niveau réduit de saccharose (7,5-15 g/l), du Gelrite (2-3 g/l) comme agent gélifiant, et de la 6-benzylaminopurine (BAP) seule (1-2 mg/l), ou en combinaison avec 1 mg/l d'acide indole-3-butyrique (IBA) et/ou 1 mg/l d'acide α-naphtalèneacétique (NAA) en tant que régulateur de croissance végétale (PGR).

5. Procédé selon l'une quelconque des revendications 1 à 4, dans lequel l'inoculation des graines sur le milieu de germination des graines est réalisée en plaçant les graines sur le milieu, l'hile des graines individuelles étant positionné au-dessus du milieu, dans lequel on laisse les graines germer pendant 2-3 semaines dans l'obscurité à 25°C.

6. Procédé selon l'une quelconque des revendications 1 à 5, qui inclut l'imbibition des graines avec une solution antioxydante avant l'étape de germination des graines, conduisant ainsi à un meilleur développement des semis par rapport à des semis dérivés de graines imbibées d'eau distillée ou de graines non imbibées, où la solution antioxydante est une solution d'acide ascorbique contenant 100-200 mg/l d'acide ascorbique, et où le temps d'imbibition est d'environ 12-24 heures.

7. Procédé selon l'une quelconque des revendications 1 à 6, dans lequel le développement des semis inclut l'incubation des cultures de semis dans des conditions contrôlées à environ 25°C, pendant une photopériode de 12-16 heures et à une intensité lumineuse d'environ 50-90 µmol.m⁻².s⁻¹, et dans lequel le développement des semis s'effectue pendant environ 4 à environ 7 semaines.

8. Procédé selon l'une quelconque des revendications 1 à 7, dans lequel
(a) l'obtention d'explants de pousses aseptiques à partir des semis s'effectue par le retrait mécanique de la racine en dessous de la plaque basale des semis ; et/ou
(b) lorsque le soulèvement de la dominance apicale d'un explant de pousse s'effectue par un retrait mécanique du dôme apical, une gaine foliaire de l'explant de pousse est gardée, et dans lequel le retrait mécanique du dôme apical est réalisé en pratiquant une entaille au-dessus d'une plaque basale des semis.

9. Procédé selon la revendication 8, dans lequel le retrait de la racine d'un semis et le retrait du dôme apical d'un explant de pousse sont réalisés alors que le semis et/ou la pousse sont immergés dans une solution antioxydante, ladite solution antioxydante comprenant :
(a) une solution d'acide ascorbique (100-200 mg/l) ; ou
(b) une solution d'acide ascorbique mélangée avec de la polyvinylpyrrolidone (PVP), empêchant ainsi le brunissement des explants en culture.

10. Procédé selon l'une quelconque des revendications 1 à 9, dans lequel l'initiation du développement de pousses multiples sur l'explant de pousse ayant le dôme apical retiré est réalisée et est effectuée en cultivant l'explant de pousse ayant le dôme apical retiré sur un milieu de base MS contenant des vitamines, du saccharose non dilué (30 g/l), du Gelrite (2-3 g/l) et au moins un régulateur de croissance végétale inducteur de pousses, dans lequel le régulateur de croissance végétale est la 6-benzylaminopurine (BAP), seule (1-4 mg/l) ou en combinaison avec 1 mg/l d'acide indole-3-butyrique (IBA), et/ou en combinaison avec 1 mg/l d'acide a-naphtalèneacétique (NAA), ou en combinaison avec 1 mg/l de kinétine.

11. Procédé selon l'une quelconque des revendications 1 à 10, dans lequel l'initiation des racines adventives s'effectue en cultivant l'explant de pousse sur un milieu d'induction de racines, ledit milieu d'induction de racines étant un milieu de culture MS solide ou liquide.

12. Procédé selon la revendication 11, dans lequel soit
(a) on utilise un milieu solide d'induction de racines qui comprend un milieu de culture de base MS, du PGR auxine (1-5 mg/l d'IBA et/ou de NAA), du Gelrite (2-3 g/l) et 30 g/l de saccharose ; soit
(b) on utilise un milieu liquide d'induction de racines qui n'est constitué que d'un milieu de culture de base MS et de PGR auxine (1-5 mg/l d'IBA et/ou de NAA).

13. Procédé selon l'une quelconque des revendications 1 à 12, dans lequel la croissance des pousses et le développement des racines sont tous deux induits simultanément sur l'explant de pousse ayant le dôme apical retiré, et dans lequel la croissance des pousses et l'induction du développement des racines simultanées s'effectuent en cultivant l'explant de pousse dans un milieu de culture contenant des combinaisons de 1-2 mg/l de PGR inducteur de pousses (BAP) et de 1-2 mg/l de PGR inducteur de racines (NAA).

14. Procédé selon l'une quelconque des revendications 1 à 13, dans lequel l'initiation du développement ou de l'induction de pousses multiples en cultivant directement les graines scarifiées et stérilisées est réalisée, et est effectuée sur un milieu de germination des graines comprenant un milieu de base MS, des vitamines, du saccharose dilué de moitié (15 g/l), 2-3 g/l de Gelrite et de la 6-benzylaminopurine (BAP), seule (1-2 mg/l) ou en combinaison avec 1 mg/l d'acide indole-3-butyrique (IBA) et/ou 1 mg/l d'acide α-naphtalèneacétique (NAA).

15. Procédé in vitro pour rendre une graine de *Strelitzia* apte à la germination, ledit procédé comprenant les étapes séquentielles consistant à :
(a) scarifier chimiquement les graines de *Strelitzia* ;
(b) ensuite, effectuer une stérilisation en surface des graines scarifiées ;
(c) imbiber les graines scarifiées et stérilisées d'une solution antioxydante ; et
(d) placer les graines scarifiées et stérilisées sur un milieu de germination des graines, un hile des graines étant positionné au-dessus du milieu, et laisser germer les graines.
